# EUROPÄISCHE PATENTSCHRIFT

(11) **EP 0 528 245 B1**
(45) Veröffentlichungstag und Bekanntmachung des Hinweises auf die Patenterteilung: **12.11.1997**
(21) Anmeldenummer: 92113197.5
(22) Anmeldetag: 03.08.1992
(51) Int. Cl.: C07D 261/08, C07D 261/10, C07D 231/12, C07F 9/653, C07D 207/32, A01N 43/76, A01N 43/56, A01N 43/36, C07D 413/04, C07F 9/6503, C07F 9/572

(54) **Alpha-Arylacrylsäurederivate, ihre Herstellung und Verwendung zur Bekämpfung von Schädlingen und Pilzen**
Alpha-arylacrylic and derivatives, their preparation and their use as pesticides and fungicides
Dérivés d'acides alpha-arylacryliques, leur préparation et leur utilisation comme pesticides et fongicides

(30) Priorität: 16.08.1991 DE 4126994
(43) Veröffentlichungstag der Anmeldung: 24.02.1993
(73) Patentinhaber: BASF Aktiengesellschaft, 67063 Ludwigshafen (DE)
(72) Erfinder: Kirstgen, Reinhard, Dr., W-6730 Neustadt (DE); Theobald, Hans, Dr., W-6703 Limburgerhof (DE); Koenig, Hartmann, Dr., W-6703 Limburgerhof (DE); Harreus, Albrecht, Dr., W-6700 Ludwigshafen (DE); Oberdorf, Klaus, Dr., W-6900 Heidelberg (DE); Kardorff, Uwe, Dr., W-6800 Mannheim 1 (DE); Harries, Volker, Dr., W-6710 Frankenthal (DE); Lorenz, Gisela, Dr., W-6730 Neustadt (DE); Ammermann, Eberhard Dr., W-6148 Heppenheim (DE)

(56) Entgegenhaltungen:
- EP-A- 0 034 754
- EP-A- 0 087 953
- EP-A- 0 178 826
- EP-A- 0 378 755
- EP-A- 0 402 246
- JOURNAL OF THE CHEMICAL SOCIETY, PERKIN TRANSACTIONS 1 1989, LETCHWORTH GB Seiten 2047 - 2057 MICHAEL J. CRIMMIN ET AL 'The chemistry of pseudomonic acid. Part10.Preparation of heterocyclic derivatives'
- JOURNAL OF THE CHEMICAL SOCIETY, PERKIN TRANSACTIONS 1 1982, LETCHWORTH GB Seiten 2983 - 2987 BARALDI PIER GIOVANNI ET AL 'Asymmetric synthesis of a beta-ketol moiety via 3,5-disubstituted isoxazoles:applications to(1)-(S)-gingerol' Seite 2984,Verbindung 6b,seite 2985
- JOURNAL OF ORGANIC CHEMISTRY. Bd. 48, Nr. 7, 8. April 1983, EASTON US Seiten 1149 - 1150 LEO A. PAQUETTE ET AL 'Methodology for the synthesis of 3- acyltetramic acid'

## Beschreibung

Die vorliegende Erfindung betrifft α-Arylacrylsäurederivate der allgemeinen Formel I 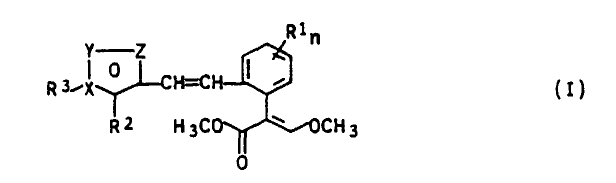 in der die Substituenten folgende Bedeutung haben:
- X: C, N
- Y: CR⁴, N, O, S
- Z: CR⁵, N, O, S, wobei Y und Z nicht gleichzeitig O, S oder O und S bedeuten,
- n: 0 bis 4
- R¹: Nitro, Cyano, Halogen,
C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio,
oder, für den Fall, daß n 2, 3 oder 4 bedeutet, zwei benachbarte Substituenten R¹ zusammen eine 1,3-Butadien-1,4-diylgruppe bedeuten, welche ein bis vier Halogenatome und oder ein oder zwei der folgenden Gruppen tragen kann: Nitro, Cyano, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
- R²: Cyano, Dimethylamino, Nitro, Halogen, C₁-C₄-Alkyl, partiell oder vollständig halogeniertes C₁-C₄-Alkyl, C₁-C₄-Alkoxycarbonyl,
- R²: bedeutet zusätzlich Wasserstoff, wobei dann R³ einen heterocyclischen oder heteroaromatischen Rest bedeutet oder R⁴ oder R⁵ verschieden von Wasserstoff sind;
- R³: Wasserstoff;
Alkyl mit bis zu 12 Kohlenstoffatomen, welches partiell oder vollständig halogeniert sein kann und welches desweiteren einen bis vier der folgenden Reste tragen kann: Cyano, Cyanato, Thiocyanato, Nitro und C₁-C₆-Alkoxy;
C₃-C₈-Cycloalkyl, C₅-C₈₋Cycloalkenyl, C₅-C₈-Cycloalkdienyl oder ein 3-bis 6-gliedriger, gesättigter oder partiell ungesättigter Heterocyclus, welcher ein bis drei Heteroatome, ausgewählt aus einer Gruppe bestehend aus zwei Stickstoffatomen, zwei Sauerstoff- und zwei Schwefelatomen oder aus einer Gruppe bestehend aus drei Sauerstoff- und Schwefelatomen, enthält, wobei diese cyclischen Gruppen noch einen bis drei der folgenden Reste tragen können: Cyano, Dimethylamino, Nitro, Halogen, C₁-C₆-Alkyl, C₁-C₄-Alkoxy, C₁-C₄-Alkylthio und C₃-C₈-Cycloalkyl, und wobei die Heterocyclen noch zusätzlich soviele Halogenatome tragen können, daß die Gesamtzahl der Reste 4 oder 5 beträgt;
oder ein ein- oder zweikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann, wobei diese Gruppen ein bis fünf Halogenatome und/oder ein bis vier der folgenden Reste tragen können: Cyano, Cyanato, Thiocyanato, Nitro, Amino, Hydroxy, Carboxyl, C₁-C₆-Alkyl, partiell oder vollständig halogeniertes C₁-C₄-Alkyl, C₁-C₆-Alkoxy, partiell oder vollständig halogeniertes C₁-C₄-Alkoxy, C₁-C₆-Alkylthio, C₃-C₈-Cycloalkyl, C₁-C₆-Alkoxycarbonyl, Phenyl oder Phenoxy, wobei die letztgenannten Phenylringe ihrerseits ein bis drei der folgenden Substituenten tragen können: Cyano, Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy, und C₁-C₄-Alkylthio, und wobei diese Phenylringe zusätzlich noch soviele Halogenatome tragen können, daß die Gesamtzahl der Reste 4 oder 5 beträgt;
- R⁴,R⁵: Wasserstoff, Cyano, Dimethylamino, Nitro, C₁-C₄-Alkyl, partiell oder vollständig halogeniertes C₁-C₄-Alkyl, C₁-C₆-Alkoxycarbonyl.

Außerdem betrifft die Erfindung Verfahren zur Herstellung dieser Verbindungen und sie enthaltende Mittel, sowie Verfahren zur Bekämpfung von Schädlingen aus der Klasse der Insekten, Spinnentiere und Nematoden, und Pilzen im Pflanzen- und Materialschutz.

Aus der Literatur sind α-Arylacrylsäurederivate als Fungizide (EP-A 203 606, EP-A 229 974), als Insektizide und Fungizide (EP-A 178 826, EP-A 378 755) und als Insektizide (EP-A 256 667, EP-A 335 519) bekannt.

Aufgabe der vorliegenden Erfindung waren neue wirksame Insektizide und Fungizide.

Demgemäß wurden die eingangs definierten α-Arylacrylsäurederivate I gefunden. Außerdem wurden Verfahren zur Herstellung dieser α-Arylacrylsäurederivate und sie enthaltende Mittel sowie Verfahren zu ihrer Verwendung gefunden.

Bedingt durch die beiden -C=C-Gruppen im Molekül können die neuen Verbindungen in Form von E(Z-Isomeren auftreten. Sowohl die verschiedenen einzelnen Isomeren als auch ihre Mischungen sind biologisch wirksam und werden von der Erfindung umfaßt. Bevorzugt sind Verbindungen, in denen beide -C=C-Gruppen in der E-Konfiguration vorliegen.

Die α-Arylacrylsäurederivate I sind auf verschiedenen beispielsweise in der eingangs zitierten Literatur beschriebenen Wegen zugänglich. Besonders vorteilhaft erhält man sie nach den im nachfolgenden beschriebenen Verfahren A und B.

### Verfahren A:

Man erhält die α-Arylacrylsäurederivate der Formel I beispielsweise dadurch, daß man ein Triphenylphosphoniumsalz oder einen Phosphonsäureester der allgemeinen Formel II (z.B. gemäß EP-A 203 606; J. Am. Chem. Soc. 83, 1732 (1961)) in einem inerten organischen Lösungsmittel in Gegenwart einer Base mit einem Benzaldehyd der allgemeinen Formel III umsetzt.

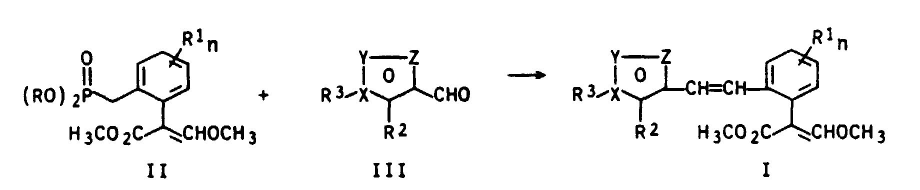

R in der Formel II steht dabei für eine C₁-C₈-Alkylgruppe wie insbesondere Methyl, Ethyl, Propyl und 1-Methylethyl.

Die Umsetzung erfolgt im allgemeinen bei Temperaturen von -30 bis 60°C, vorzugsweise von 0 bis 40°C.

Als Lösungsmittel eignen sich beispielsweise Diethylether, Benzol, Toluol, Tetrahydrofuran, Dimethoxyethan, Methanol, Ethanol und Dimethylformamid.

Insbesondere kommen Tetrahydrofuran und Dimethylformamid in Betracht.

Als Basen finden in diesem Verfahren n-Butyllithium, Natriumhydrid, Natriummethanolat, Kalium-t.-butylat, Natrium-t.-amylat, Lithiumdimethylamid und Lithiumbistrimethylsilylamid Verwendung.

Die Herstellung der benötigten Vorprodukte ist beispielsweise in der eingangs zitierten Literatur beschrieben.

Ein Triphenylphosphoniumsalz hat beispielsweise die folgende Formel

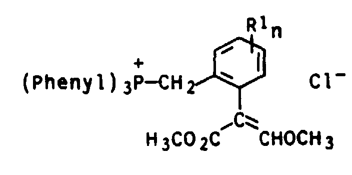

### Verfahren B:

Man erhält die α-Arylacrylsäurederivate der Formel I beispielsweise auch dadurch, daß man ein Triphenylphosphoniumsalz oder einen Phosphonsäureester der allgemeinen Formel IV in einem inerten organischen Lösungsmittel in Gegenwart einer Base mit dem Benzaldehyd V umsetzt.

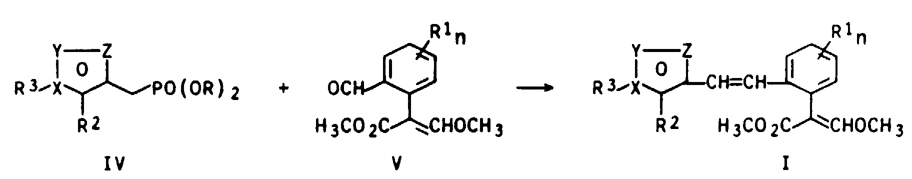

R in der Formel IV steht dabei für eine C₁-C₈-Alkylgruppe wie insbesondere Methyl, Ethyl, Propyl und 1-Methylethyl.

Die Umsetzung erfolgt im allgemeinen bei Temperaturen von -30 bis 60°C, vorzugsweise von 0 bis 40°C.

Als Lösungsmittel und als Basen eignen sich im allgemeinen und im besonderen die bei Verfahren A genannten.

Ein Triphenylphosphoniumsalz hat beispielsweise die folgende Formel

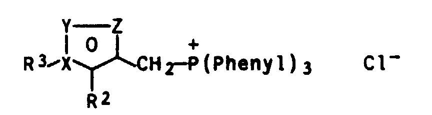

Im Hinblick auf die bestimmungsgemäße Verwendung der Verbindungen I in insektiziden und fungiziden Mitteln kommen als Substituenten insbesondere folgende Reste in Betracht:
X
   C, N
Y
   CH, CR⁴, N, O, S
Z
   CH, CR⁵, N, O, S
n
   0 bis 4
R¹
   Nitro; Cyano;
   Halogen wie Fluor, Chlor, Brom und Jod, vorzugsweise Fluor, Chlor und Brom, insbesondere Fluor und Chlor;
   verzweigtes oder unverzweigtes C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 1-Methylethyl und 1,1-Dimethylethyl, insbesondere Methyl und 1,1-Dimethylethyl;
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy und 1,1-Dimethylethoxy, insbesondere Methoxy;
   partiell oder vollständig halogeniertes C₁-C₄-Alkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Dichlormethyl, Trichlormethyl und Trifluormethyl, insbesondere Trifluormethyl;
   partiell oder vollständig halogeniertes C₁-C₄-Alkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy, 1,1,2,2-Tetrafluorethoxy und Pentafluorethyloxy, vorzugsweise Trichlormethoxy, Difluormethoxy, Trifluormethoxy, Chlordifluormethoxy und 1,1,2,2-Tetrafluorethoxy insbesondere Difluormethoxy und 1,1,2,2-Tetrafluorethoxy;
   oder C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio und Ethylthio, insbesondere Methylthio;
   oder, für den Fall, daß n für 2, 3 oder 4 steht, eine 1,3-Butadien-1,4-diylgruppe, welche ein bis vier Halogenatome wie Fluor, Chlor, Brom und Jod, vorzugsweise Fluor und Chlor, insbesondere Chlor und/oder ein oder zwei der folgenden Gruppen tragen kann:
   Nitro, Cyano,
   unverzweigtes oder verzweigtes C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl und Ethyl, insbesondere Methyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy, insbesondere Methoxy,
   partiell oder vollständig halogeniertes C₁-C₄-Alkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl,
   partiell oder vollständig halogeniertes C₁-C₄-Alkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethoxy und 1,1,2,2-Tetrafluorethoxy, insbesondere Difluormethoxy,
   oder C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio;
R²
   Nitro; Cyano; Dimethylamino;
   Halogen wie vorstehend genannt, vorzugsweise Fluor, Chlor und Brom, insbesondere Chlor und Brom;
   verzweigtes oder unverzweigtes C₁-C₄-Alkyl wie vorstehend genannt, insbesondere Methyl, 1-Methylethyl, 1-Methylpropyl und 1,1-Dimethylethyl;
   partiell oder vollständig halogeniertes C₁-C₄-Alkyl wie vorstehend vorzugsweise und insbesondere genannt;
   unverzweigtes oder verzweigtes C₁-C₆-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Me-thylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylprop-oxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1, l-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycar-bonyl, 2-Ethylbutyloxycarbonyl, 1,1,1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise C₁-C₄-Alkoxycarbonyl, insbesondere Methoxycarbonyl, Ethoxycarbonyl und 1,1-Dimethylethoxycarbonyl;
   Alkyl mit bis zu 12 Kohlenstoffatomen in der Bedeutung R³ der allgemeinen Formel I ist geradkettig oder verzweigt, besonders C₁-C₆-Alkylgruppen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und l-Ethyl-2-methylpropyl, vorzugsweise Methyl, Ethyl, Propyl und 1-Methylethyl, insbesondere Methyl, Ethyl, Propyl, Butyl und 1,1-Dimethylethyl, wobei diese Gruppen partiell oder vollständig halogeniert sein können.

Unter partiell oder vollständig halogenierten Alkylgruppen sind diejenigen bevorzugt, die ein bis neun Halogenatome wie vorstehend genannt, vorzugsweise Fluor, Chlor und Brom, insbesondere Chlor und Fluor, tragen.

Die genannten Alkylgruppen R³ können des weiteren einen bis vier der folgenden Reste tragen:
Cyano, Cyanato, Thiocyanato, Nitro,
unverzweigtes oder verzweigtes C₁-C₆-Alkoxy wie wie Methoxy, Ethoxy, n-Propyloxy, 1-Methylethoxy, n-Butyloxy, 1-Methylpropyloxy, 2-Methylpropyloxy, 1,1-Dimethylethyloxy, n-Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropoxy, 1-Ethylpropyloxy, n-Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise C₁-C₄-Alkoxy, insbesondere C₁-C₂-Alkoxy;

R³ in der allgemeinen Formel I steht außerdem für die folgenden Cyclen:
C₃-C₈-Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl, insbesondere Cyclopropyl;
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-4-enyl, vorzugsweise Cyclopent-1-enyl, Cyclopent-3-enyl und Cyclohex-1-enyl, insbesondere Cyclopent-3-enyl;
C₅-C₈-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa1,4-dien-3-yl, Cyclohepta-1,3-dien-1-yl, Cyclohepta-1,3-dien-2-yl, Cyclohepta-1,3-dien-5-yl, Cyclohepta-1, 3-dien-6-yl, Cycloocta-1,3-dien-1-yl, Cycloocta-1,3-dien-2-yl, Cycloocta-1, 3-dien-5-yl, Cycloocta-1,3-dien-6-yl, Cycloocta-1, 4-dien-1-yl, Cycloocta-1, 4-dien-2-yl, Cycloocta-1, 4-dien-3-yl, Cycloocta-1,4-dien-6-yl und Cycloocta-1,4-dien-7-yl, vorzugsweise Cyclopenta-1,3-dien-5-yl;
3- bis 6-gliedrige, gesättigte oder partiell ungesättigte Heterocyclen, enthaltend ein bis drei Stickstofatome oder ein bis drei Heteroatome, ausgewählt aus einer Gruppe bestehend aus zwei Stickstoffatomen, zwei Sauerstoff- und zwei Schwefelatomen oder aus einer Gruppe bestehend aus drei Sauerstoff- und Schwefelatomen wie Epoxy, 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2,5-Dihydrothien-2-yl, 2,5-Dihydrothien-3-yl, 2,3-Pyrrolin-2-yl, 2,3-Pyrrolin-3-yl, 2,4-Pyrrolin-2-yl, 2,5-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, N-Morpholinyl und Dihydrochinazolinyl, insbesondere 2-Oxazolidinyl, 1,3-Dihydrooxazin-2-yl, Oxazol-2-in-2-yl, Oxiranyl, 2-Tetrahydrofuranyl, 1,3-Dithian-2-yl, 2-Tetrahydropyranyl und 1,3-Dioxolan-2-yl, zu verstehen,
wobei diese Gruppen noch ein bis drei Reste tragen können, ausgewählt aus einer Gruppe bestehend aus Halogen wie vorstehend genannt, vorzugsweise Fluor, Chlor und Brom, insbesondere Fluor und Chlor, Cyano; Nitro; Dimethylamino;
unverzweigtes oder verzweigtes C₁-C₆-Alkyl wie vorstehend im allgemeinen und im besonderen genannt;
C₁-C₄-Alkyloxy wie vorstehend im allgemeinen und im besonderen genannt;
C₁-C₄-Alkylthio wie vorstehend im allgemeinen und im besonderen genannt;
C₃-C₈-Cycloalkyl wie vorstehend im allgemeinen und im besonderen genannt; Phenyl, und wobei die Heterocyclen noch zusätzlich so viele Halogenatome wie vorstehend genannt, vorzugsweise Fluor, Chlor und Brom, insbesondere Fluor und Chlor tragen können, daß die Gesamtzahl der Reste 4 oder 5 beträgt.
Unter ggf. subst. ein- oder zweikernigen aromatischen Systemen, welche neben Kohlenstoffatomen ein bis vier Stickstoffatome oder ein oder zwei Stickstoffatome und ein Sauerstoff- oder Schwefelatom enthalten können, in der Bedeutung R der allgemeinen Formel I sind aromatische und heteroaromatische Ringsysteme mit bis zu 10 Ringgliedern, besonders Phenyl, 1-Naphthyl und 2-Naphthyl, insbesondere Phenyl,
fünfring Heteroaromaten enthaltend ein bis drei Heteroatome, ausgewählt aus einer Gruppe bestehend aus drei Stickstoffatomen und einem Sauerstoff- oder Schwefelatom, wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3yl, 1,2,4-Thiadiazol-5-yl, 1,2,4-Triazol-3-yl, 1,3,4-Triazol-2-yl, 1,3,4-Oxadiazol-2-yl, 1,3,4-Thiadiazol-2-yl, 1,3,4-Triazol-2-yl, 1,2,3-Oxadiazol-4-yl, 1,2,3-Oxadiazol-5-yl, 1,2,5-Triazol-3-yl, 1,2,3-Triazol-4-yl, 1,2,3-Triazol-5-yl, 1,2,3-Triazol-4-yl, 1,2,3,4-Thiatriazol-5-yl und 1,2,3,4-Oxatriazol-5-yl, insbesondere 3-Pyrrolyl, 3-Isoxazolyl, 5-Isoxazolyl, 2-Furyl, 2-Thienyl, 4-Oxazolyl, 4-Thiazolyl, 4-Pyrazolyl, 5-Pyrazolyl, 1,3,4-Oxadiazol-2-yl und 1,3,4-Thiadiazol-2-yl,
sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 2-Pyrimidinyl, 4-Pyrimidinyl und 1,3,5-Triazin-2-yl,
zu verstehen, wobei diese Gruppen ein bis fünf Halogenatome wie vorstehend genannt, vorzugsweise Fluor, Chlor und Brom, insbesondere Fluor und Chlor, und/oder ein bis vier der folgenden Reste tragen können:
Cyano; Cyanato; Thiocyanato; Nitro; Amino; Hydroxy; Carboxyl;
C₁-C₆-Alkyl wie vorstehend im allgemeinen und im besonderen genannt;
partiell oder vollständig halogeniertes C₁-C₄-Alkyl wie vorstehend im allgemeinen und im besonderen genannt;
C₁-C₆-Alkyloxy wie vorstehend im allgemeinen und im besonderen genannt;
partiell oder vollständig halogeniertes C₁-C₄-Alkoxy wie vorstehend im allgemeinen und im besonderen genannt;
C₁-C₆-Alkylthio wie vorstehend im allgemeinen und im besonderen genannt;
C₃-C₈-Cycloalkyl wie vorstehend im allgemeinen und im besonderen genannt;
C₁-C₆-Alkoxycarbonyl wie vorstehend im allgemeinen genannt, vorzugsweise C₁-C₄-Alkoxycarbonyl;
Phenyl, Phenoxy.

Die vorstehend genannten Phenylreste können ihrerseits ein bis drei Substituenten tragen, ausgewählt aus einer Gruppe bestehend aus Halogen wie vorstehend im allgemeinen und im besonderen genannt,
Cyano; Nitro;
C₁-C₄-Alkyl wie vorstehend im allgemeinen und im besonderen genannt;
partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt;
oder C₁-C₄-Alkylthio wie vorstehend im allgemeinen und im besonderen genannt,
und die vorstehend genannten Phenylreste können zusätzlich noch so viele Halogenatome wie vorstehend im allgemeinen und im besonderen genannt, tragen, daß die Gesamtzahl ihrer Reste 4 oder 5 beträgt.

R⁴, R⁵ bedeuten beispielsweise
Wasserstoff; Nitro; Cyano; Dimethylamino;
Halogen wie vorstehend genannt, vorzugsweise Fluor, Chlor und Brom, insbesondere Chlor und Brom;
verzweigtes oder unverzweigtes C₁-C₄-Alkyl wie vorstehend genannt, insbesondere Methyl, 1-Methylethyl, 1-Methylpropyl und 1,1-Dimethylethyl;
partiell oder vollständig halogeniertes C₁-C₄-Alkyl wie vorstehend vorzugsweise und insbesondere genannt;
verzweigtes oder unverzweigtes C₁-C₆-Alkoxycarbonyl wie vorstehend vorzugsweise und insbesondere genannt;
Im Hinblick auf ihre biologische Wirksamkeit gegen Schädlinge und Pilze sind Verbindungen der allgemeinen Struktur I bevorzugt, in denen die Substruktur 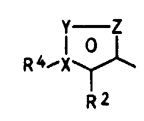 für einen der folgenden Reste steht:

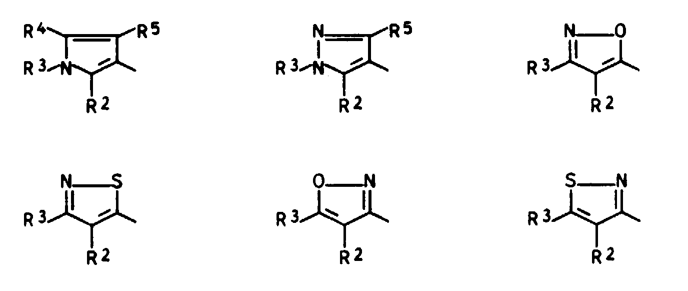

### Herstellungsbeispiel 1

### Darstellung von α-2-[2-(5-Methyl-1-phenyl-pyrazol-4-yl)-ethen-1-yl]-phenyl-β-methoxy-acrylsäuremethylester (Beispiel Nr. 2.18)

Zu einer Suspension von 0,7 g Natriumhydrid in 25 ml wasserfreiem Tetrahydrofuran wird bei Raumtemperatur eine Lösung von 7,9 g 2-(β-Methoxy-α-methoxycarbonyl-vinyl)-benzylphosphonsäuredimethylester und 5,1 g 5-Methyl-1-phenyl-pyrazol-4-yl-carboxaldehyd in 80 ml Tetrahydrofuran getropft. Dabei soll die Innentemperatur 30°C nicht übersteigen. Der Ansatz wird über Nacht weitergerührt, anschließend mit Eiswasser hydrolysiert und mit Methyl-tert.-butylether extrahiert. Die organische Phase wird über Natriumsulfat getrocknet. Nach Abziehen des Lösungsmittels wird das Rohprodukt über eine Kieselsäule chromatographiert (Laufmittel: Toluol/Ethylacetat, 9:1). Man erhält 3,1 g der Titelverbindung als farblose Kristalle. Fp.: 132-148°C.
¹H-NMR (CDCl₃, δ in ppm): 2,4 (s,3H); 3,7 (s,3H); 3,8 (s,3H); 6,85 (d,2H); 7,1-7,75 (m,10H); 7,8 (s,1H)

### Herstellungsbeispiel 2

### Darstellung von α-2-[2-(4-Chlor-3-phenyl-isoxazol-5-yl)-ethen-1-yl]-phenyl-ß-methoxy-acrylsäuremethylester (Beispiel Nr. 3.17)

Zu einer Suspension von 0,53 g Natriumhydrid in 20 ml wasserfreiem Dimethylformamid wird bei Raumtemperatur eine Lösung von 6,6 g 4-Chlor-3-phenyl-isoxazol-5-yl-methanphosphonsäuremethylester in 50 ml DMF getropft. Man rührt weitere ... min und dosiert zu dem dunkelbraunen Ansatz innerhalb von 60 min eine Lösung von 4,4 g 2-(β-Methoxy-α-methoxycarbonylvinyl)-benzaldehyd in 50 ml Dimethylformamid hinzu. Nach weiteren 3 h wird der Ansatz mit Eiswasser hydrolysiert und mit Methyl-tert.-butylether extrahiert. Die organische Phase wird über Natriumsulfat getrocknet. Nach Abziehen des Lösungsmittels verbleibt ein Feststoff. Durch verreiben mit wenig Methyl-tert.-butylether erhält man 4,0 g der Titelverbindung als farblose Kristalle. Fp.: 129-136°C.
¹H-NMR (CDCl₃, δ in ppm): 3,75 (s,3H); 3,85 (s,3H); 7,0 (d,1H); 7,2-7,9 (m,11H)

### Herstellungsbeispiel 3

### Darstellung von α-2-(2-[4-Ethyl-5-(4-Chlorphenyl)-isoxazol-3-yl]-ethen-1-yl)-phenyl-β-methoxy-acrylsäuremethylester (Beispiel Nr. 4.23)

Zu einer Suspension von 0,53 g Natriumhydrid in 20 ml wasserfreiem Dimethylformamid wird bei Raumtemperatur eine Lösung von 6,2 g 4-Ethyl-5-(4-Chlorphenyl)-isoxazol-3-yl-methanphosphonsäurediethylester in 20 ml DMF getropft. Man rührt weitere 20 min und dosiert zu dem dunkelbraunen Ansatz innerhalb von 60 min eine Lösung von 4,4 g 2-(β-Methoxy-α-methoxycarbonylvinyl)-benzaldehyd in 20 ml Dimethylformamid hinzu. Nach weiteren 3 h wird der Ansatz mit Eiswasser hydrolysiert und mit Methyl-tert.-butylether extrahiert. Die organische Phase wird über Natriumsulfat getrocknet. Nach Abziehen des Lösungsmittels wird das Rohprodukt über eine Kieselgelsäule chromatographiert (Laufmittel: Toluol). Man erhält 2,6 g der Titelverbindung als Kristalle. Fp.: 117-120°C.
¹H-NMR (CDCl₃, δ in ppm): 1,25 (t,3H); 2,7 (q,2H); 3,7 (s,3H); 3,85 (s,3H); 6,95 (d,1H); 7,1-7,8 (m,10H)

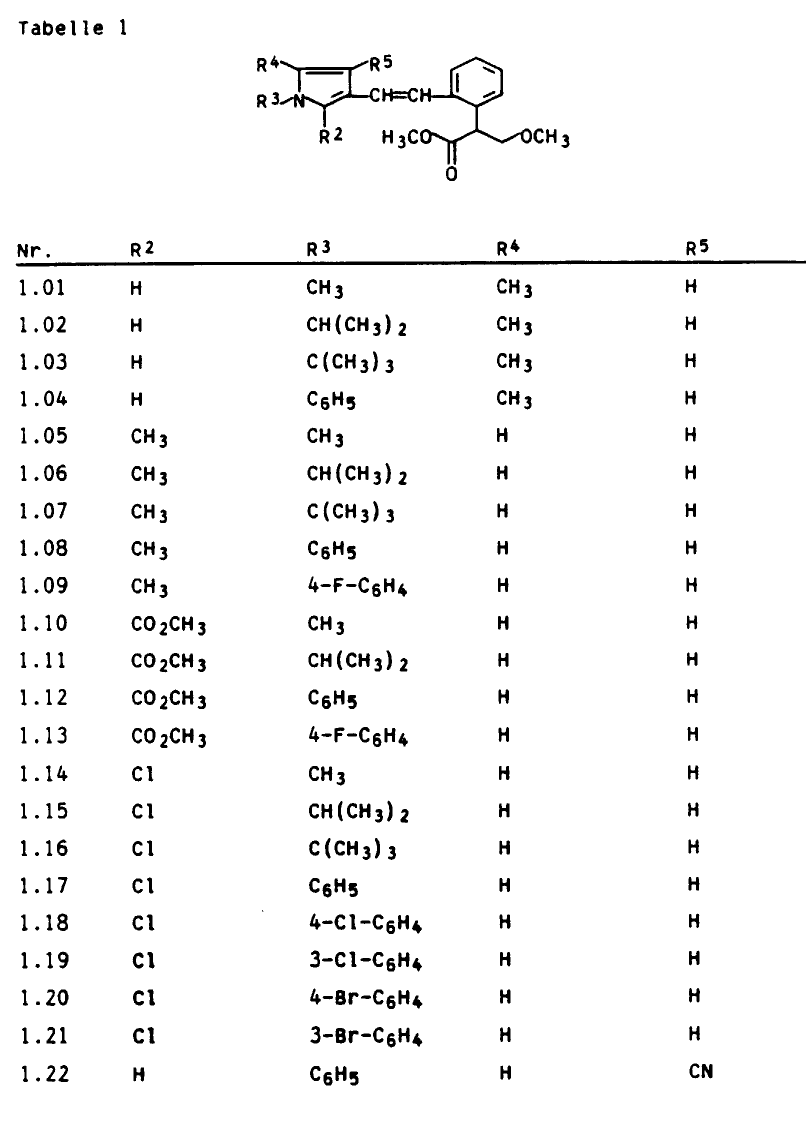

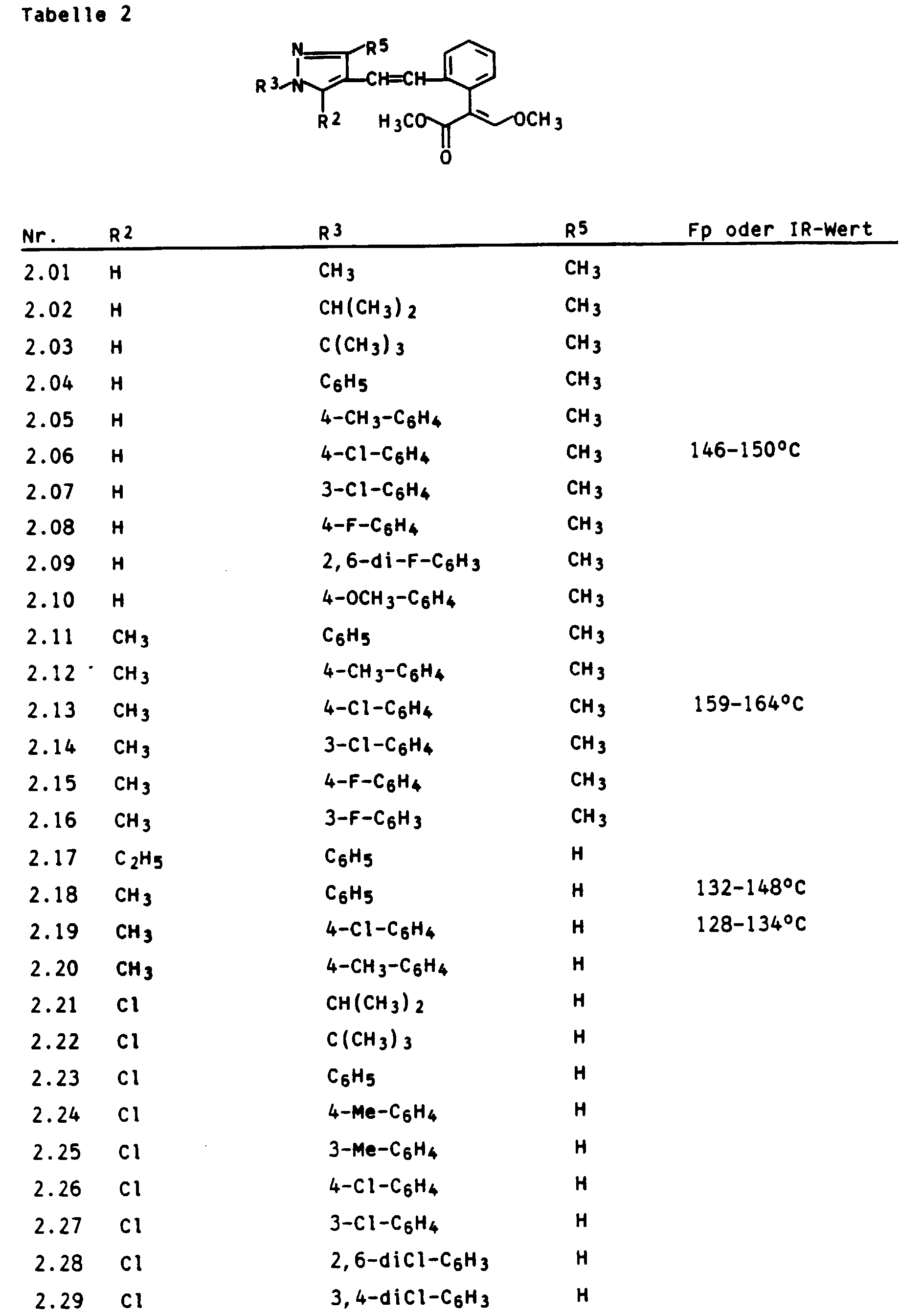

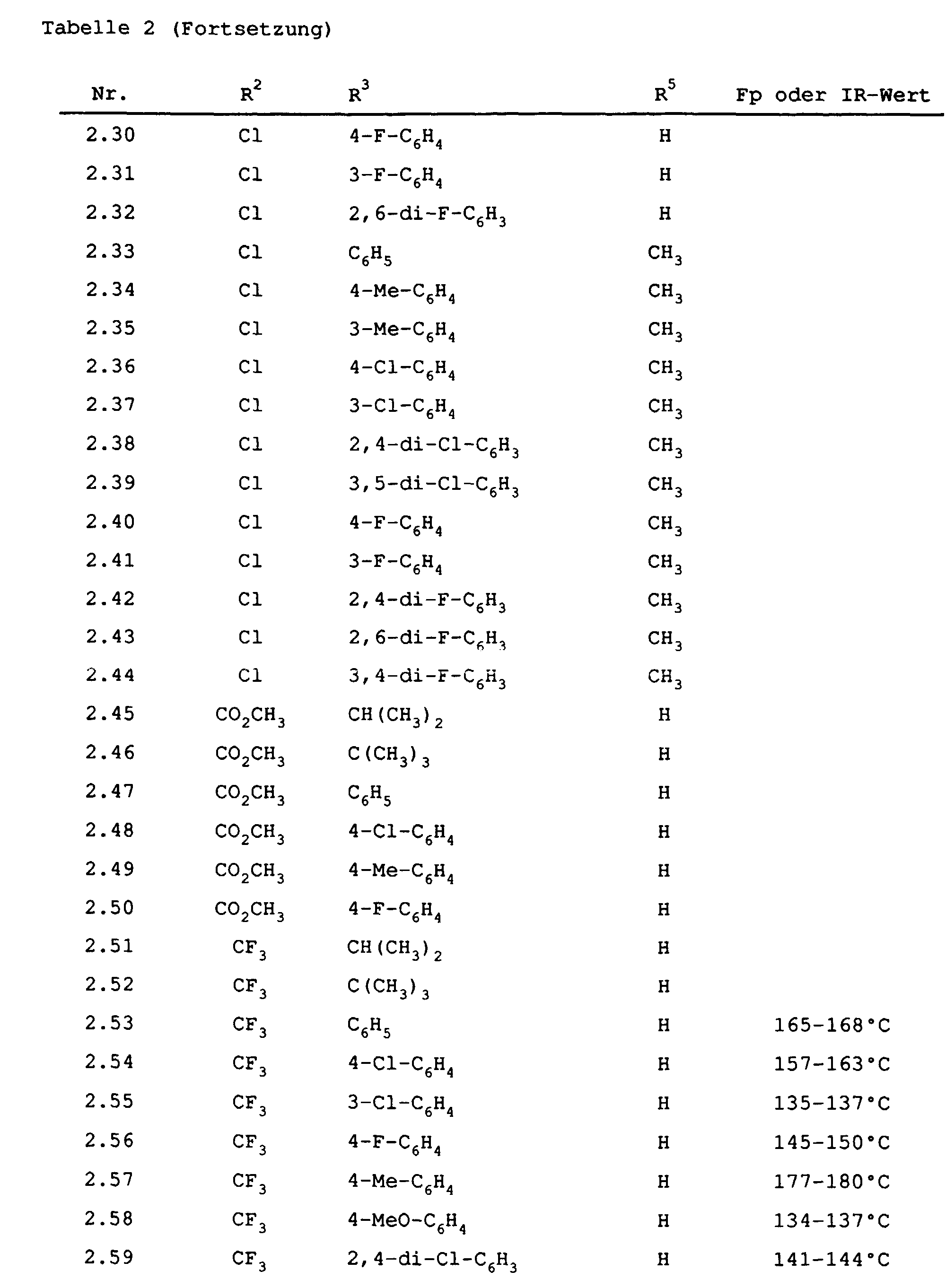

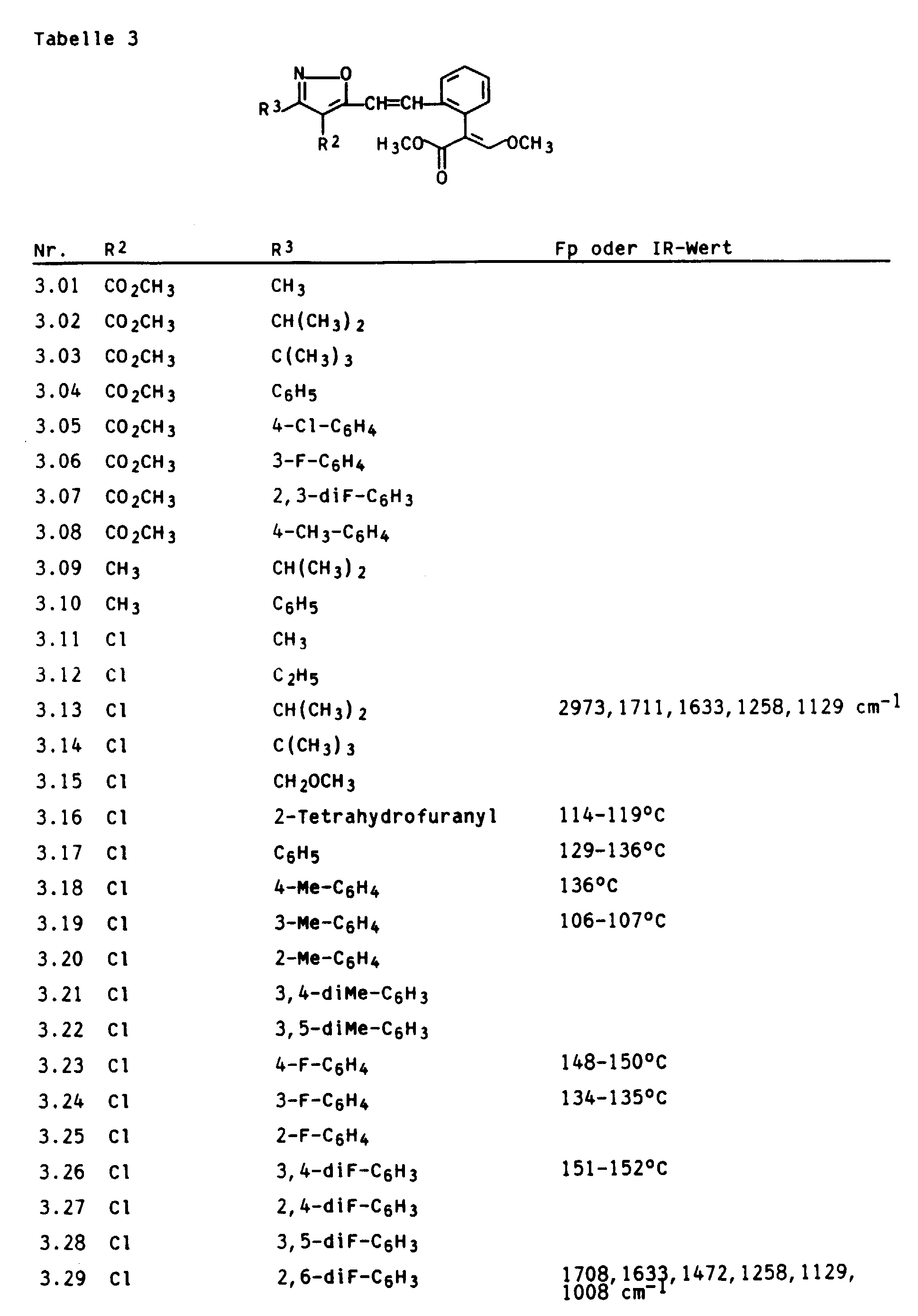

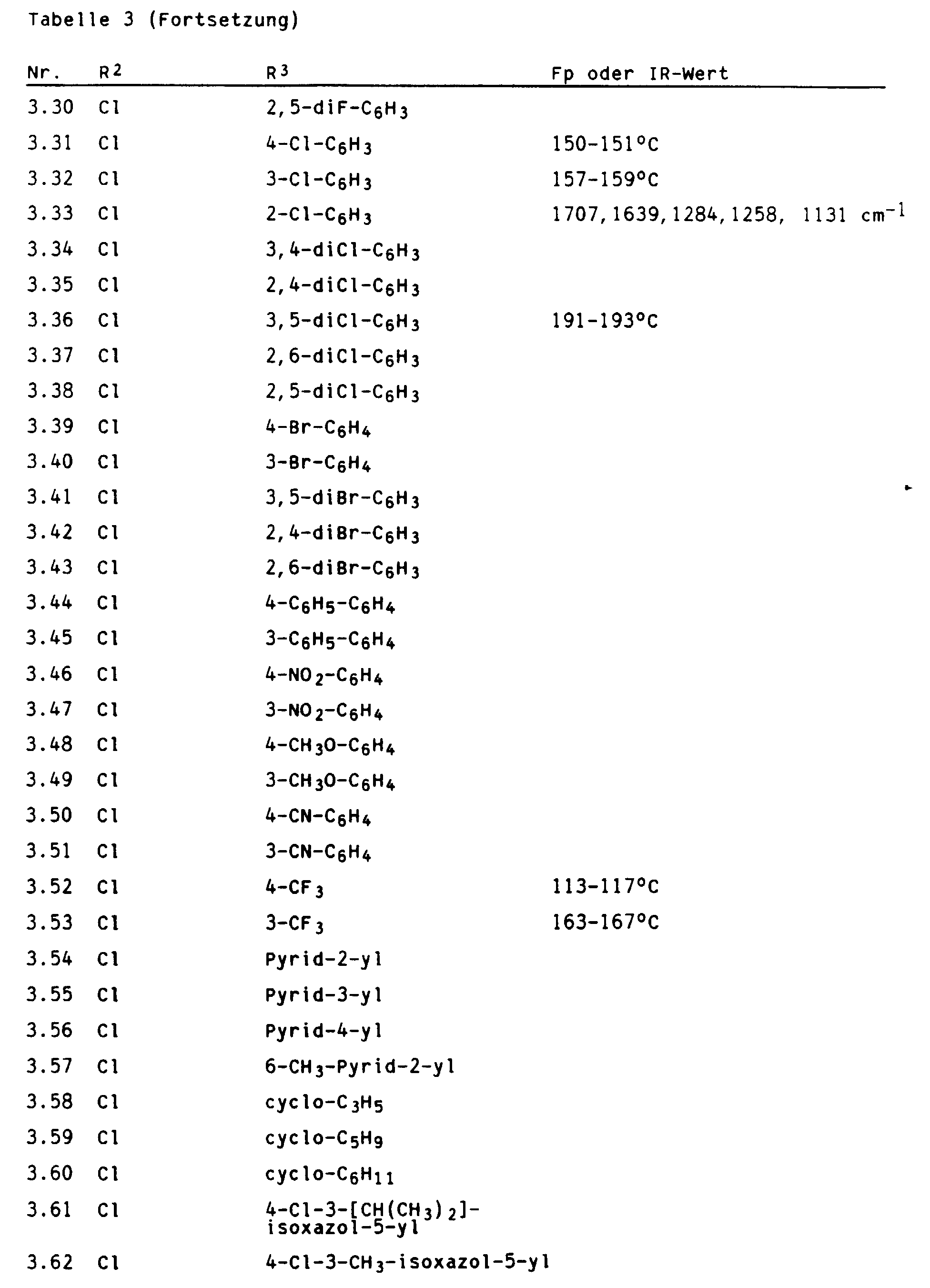

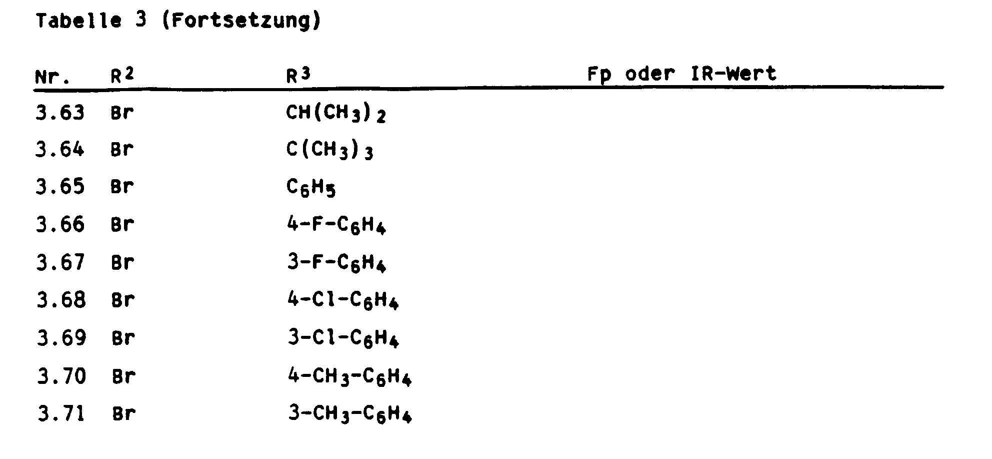

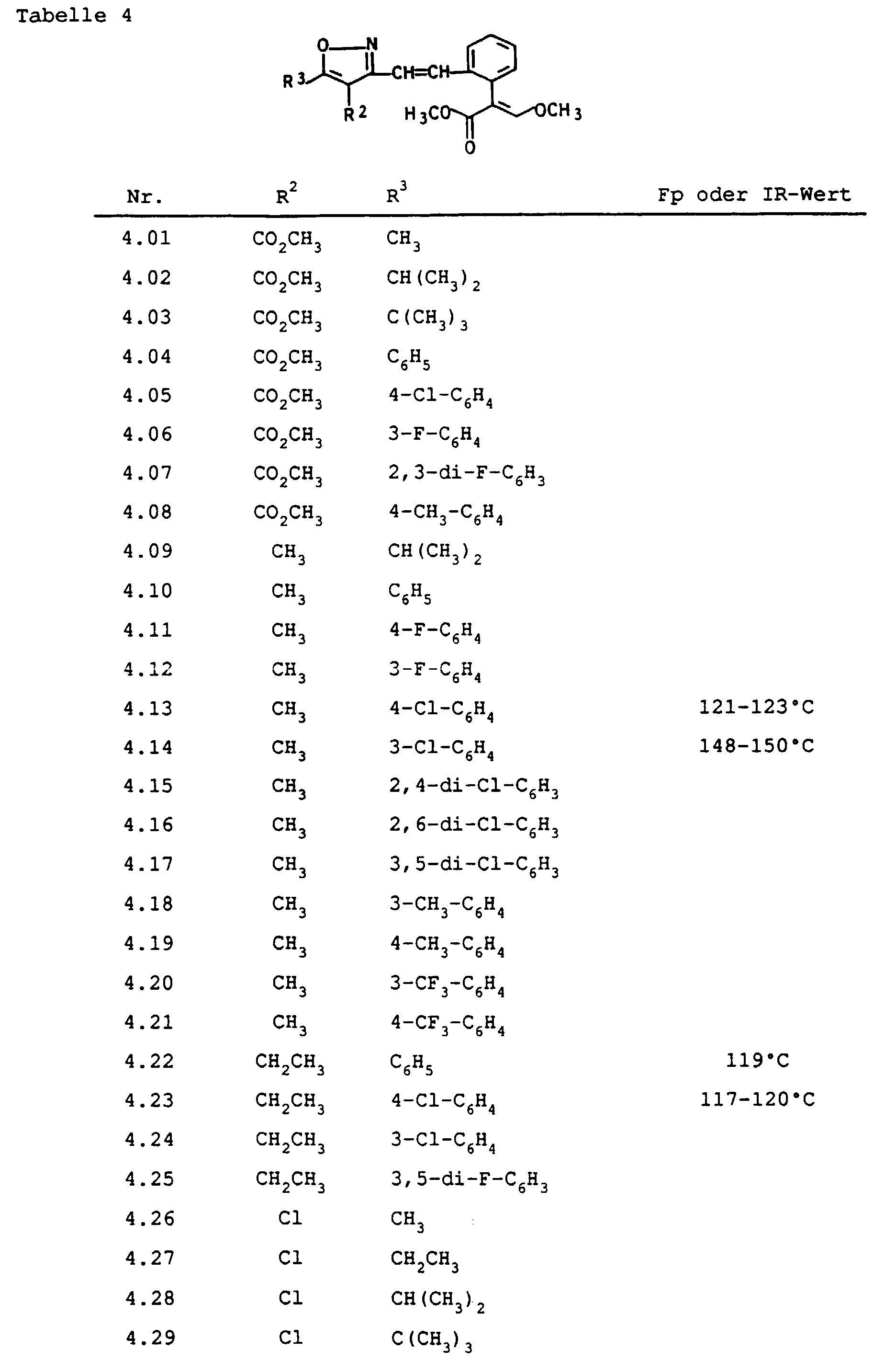

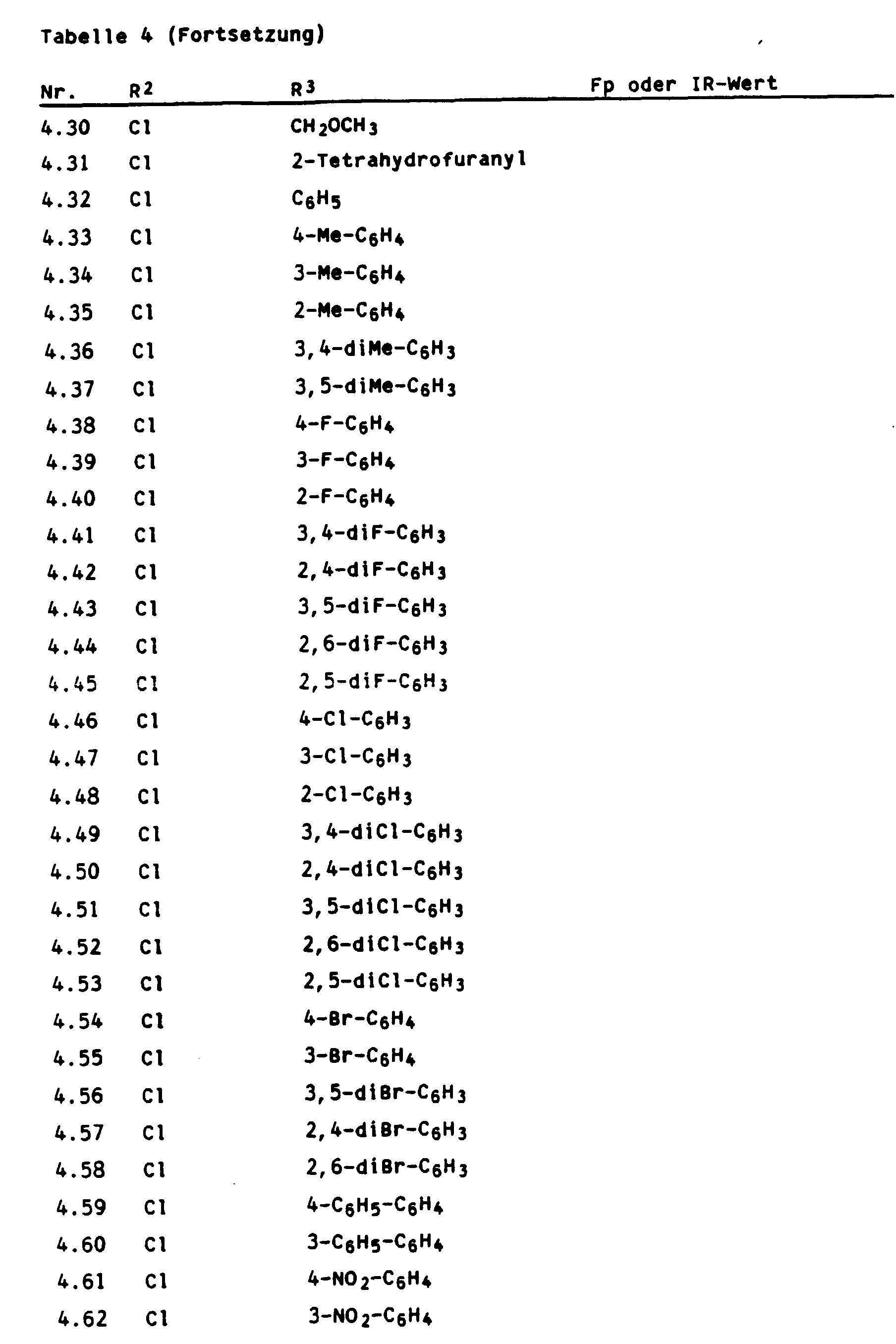

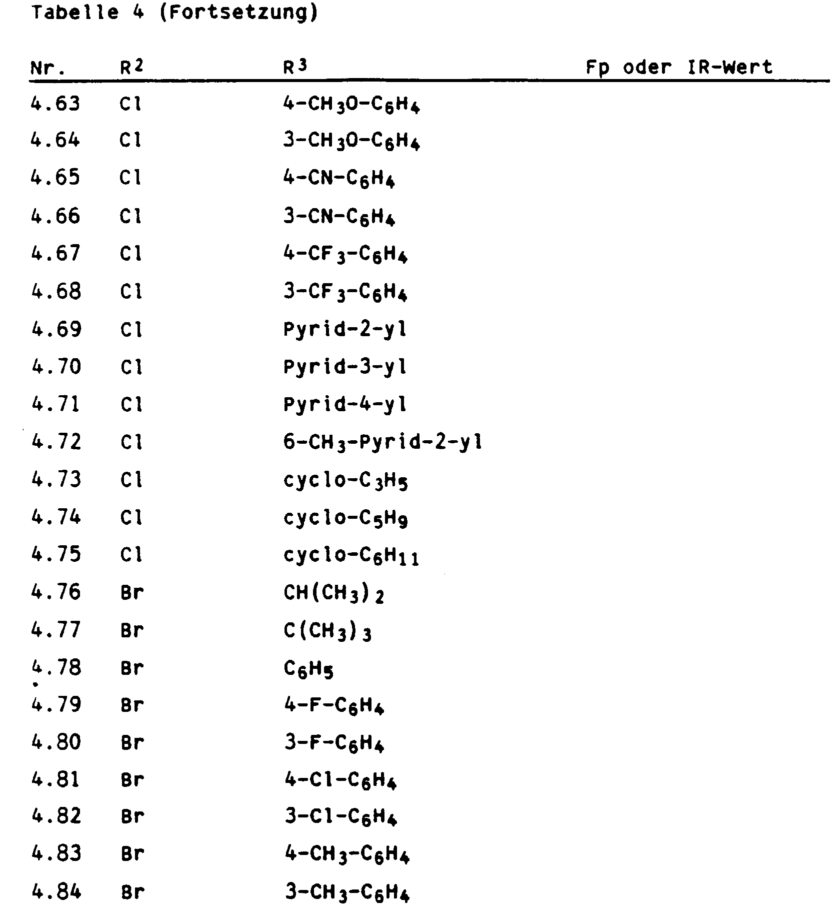

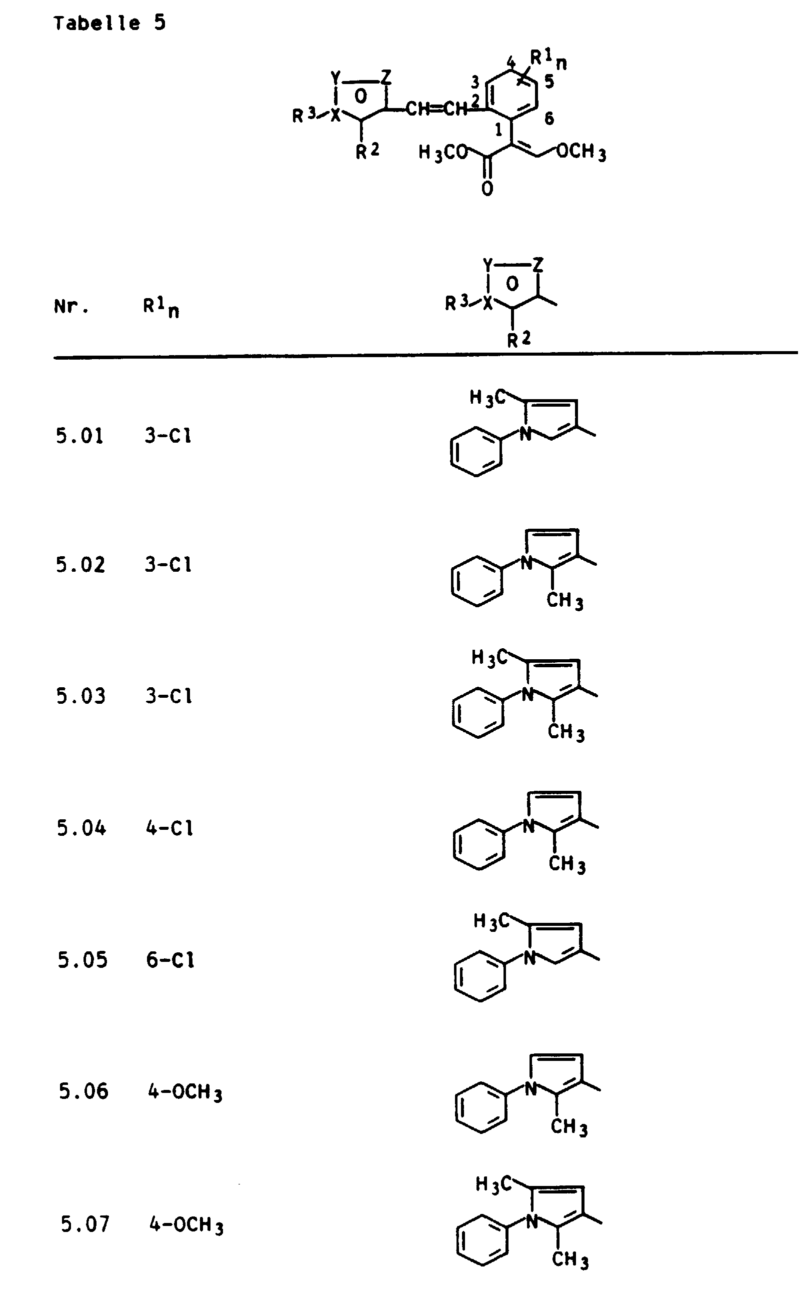

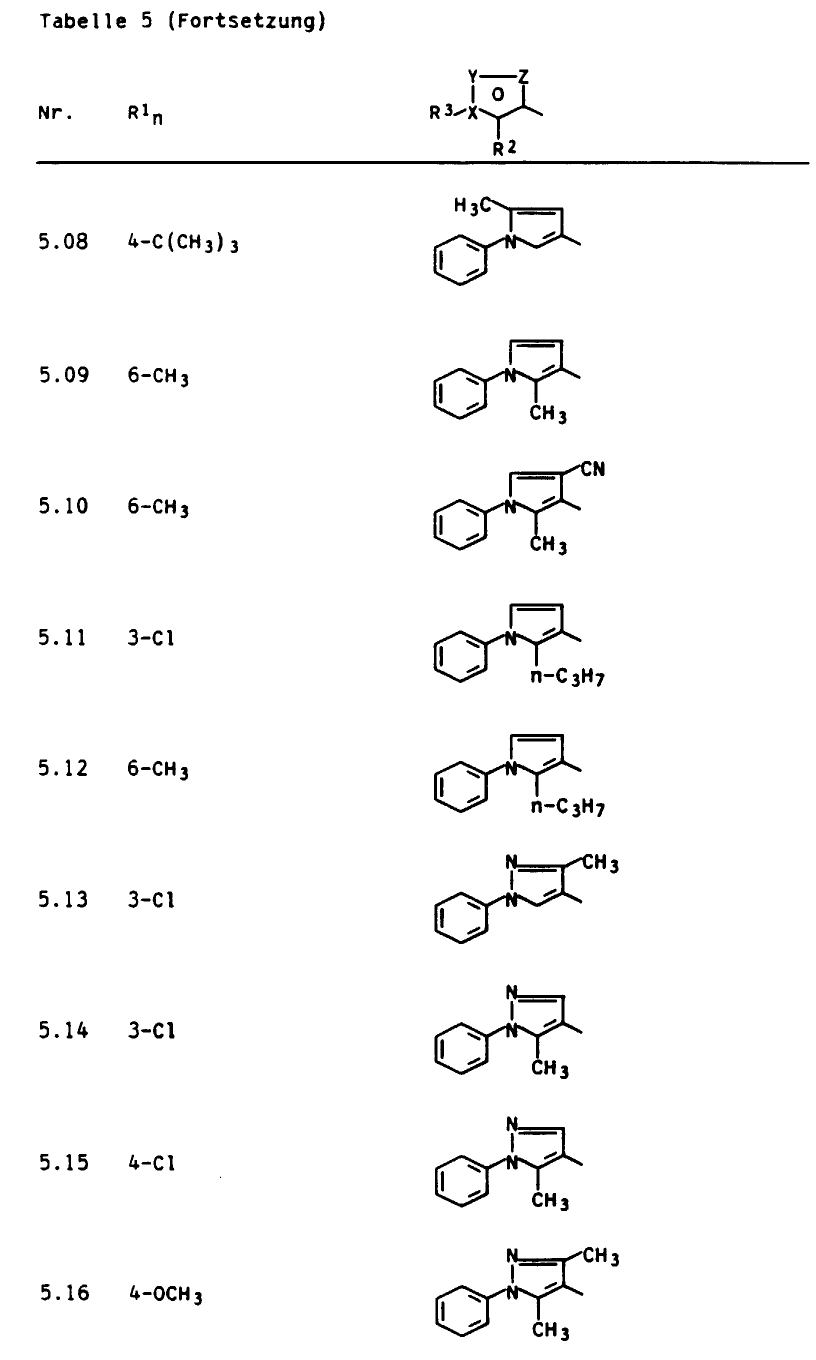

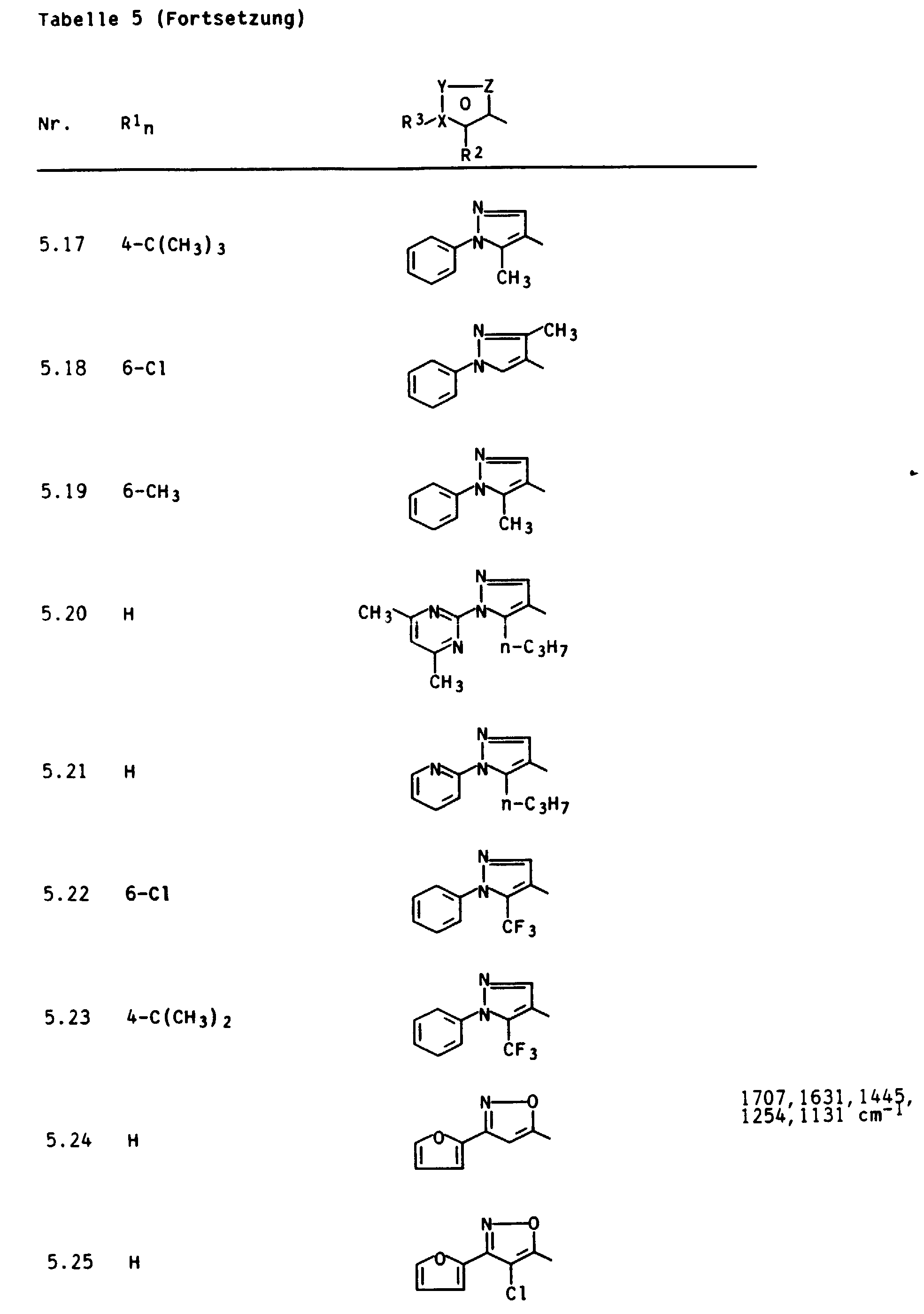

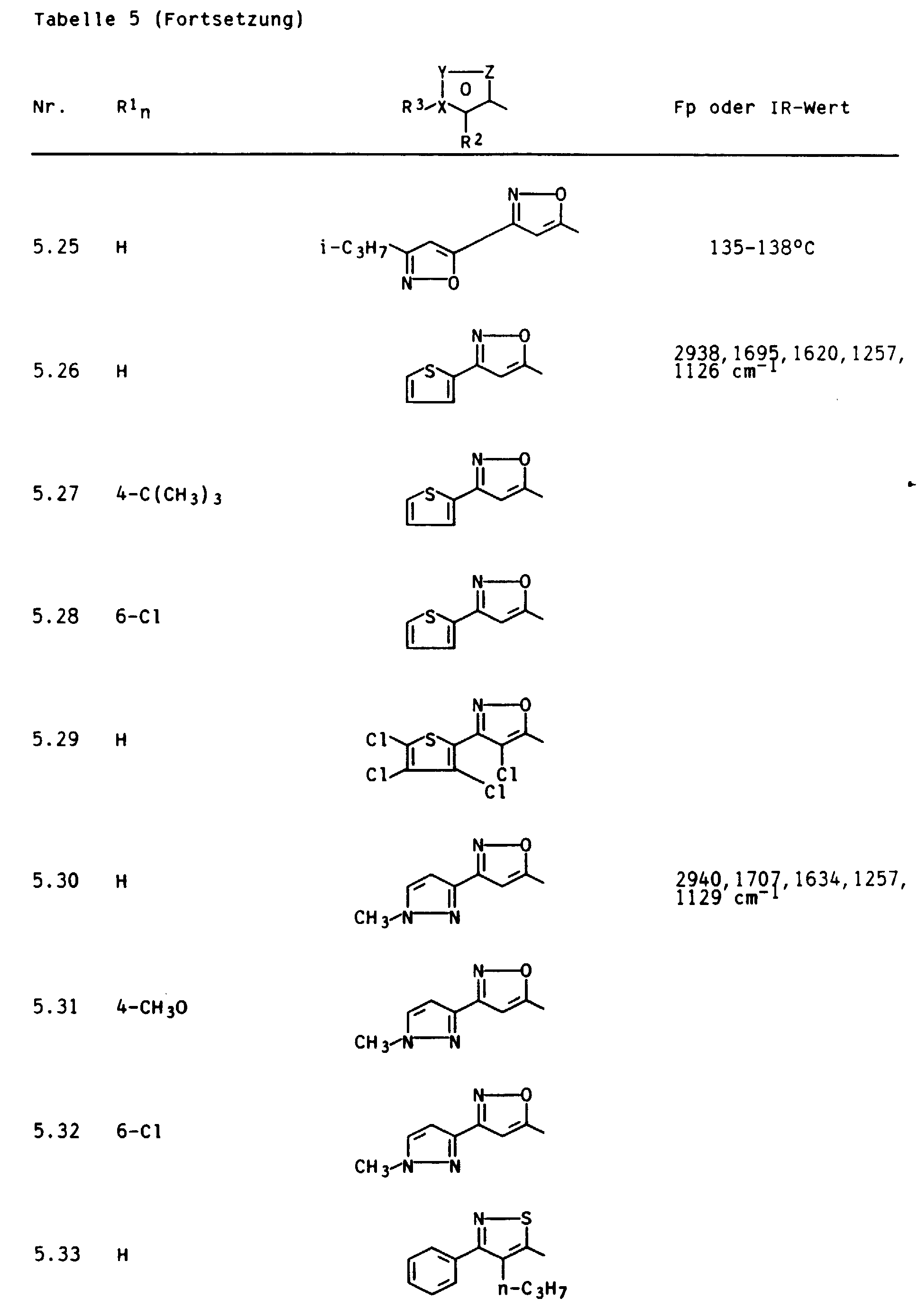

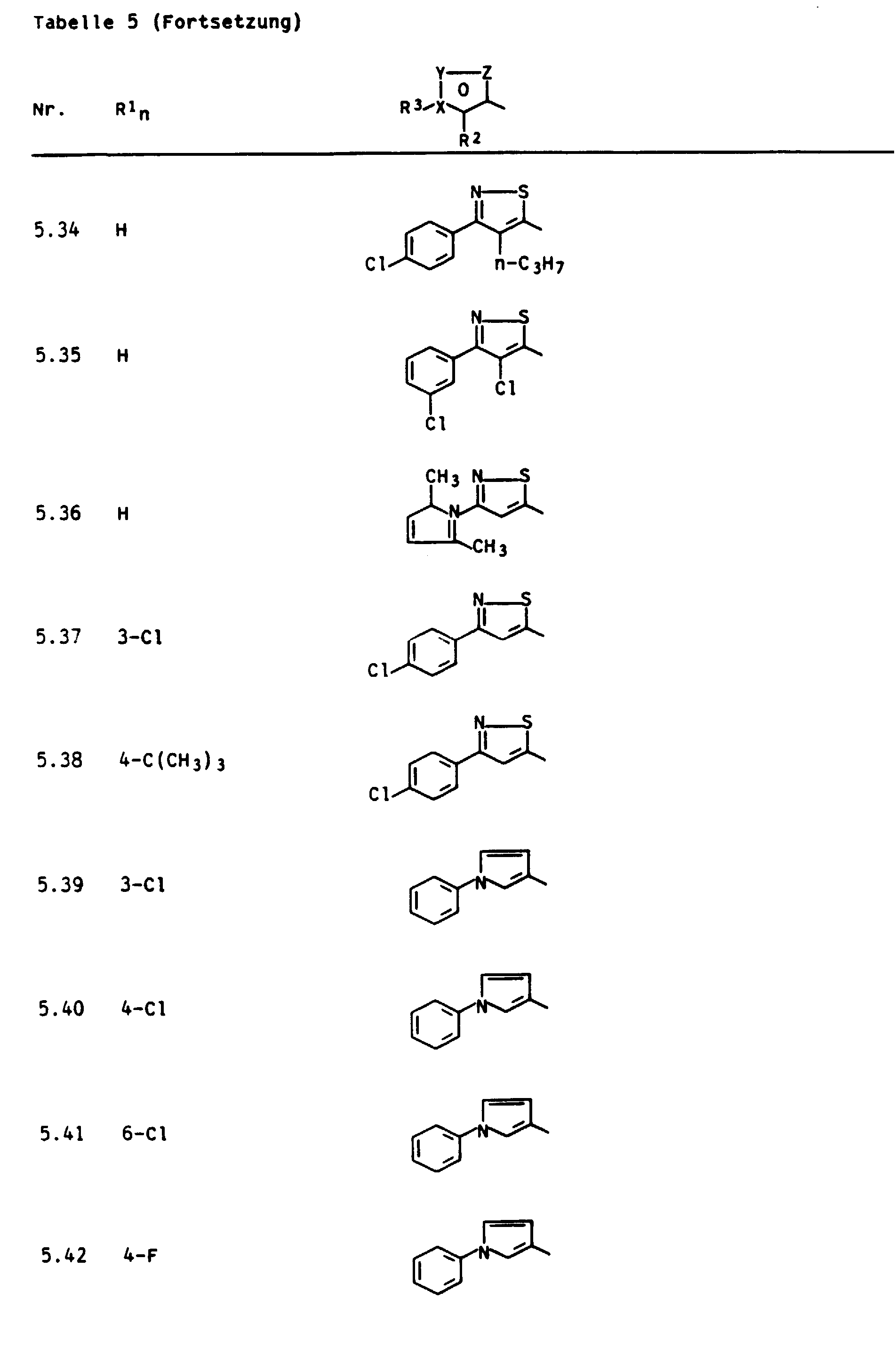

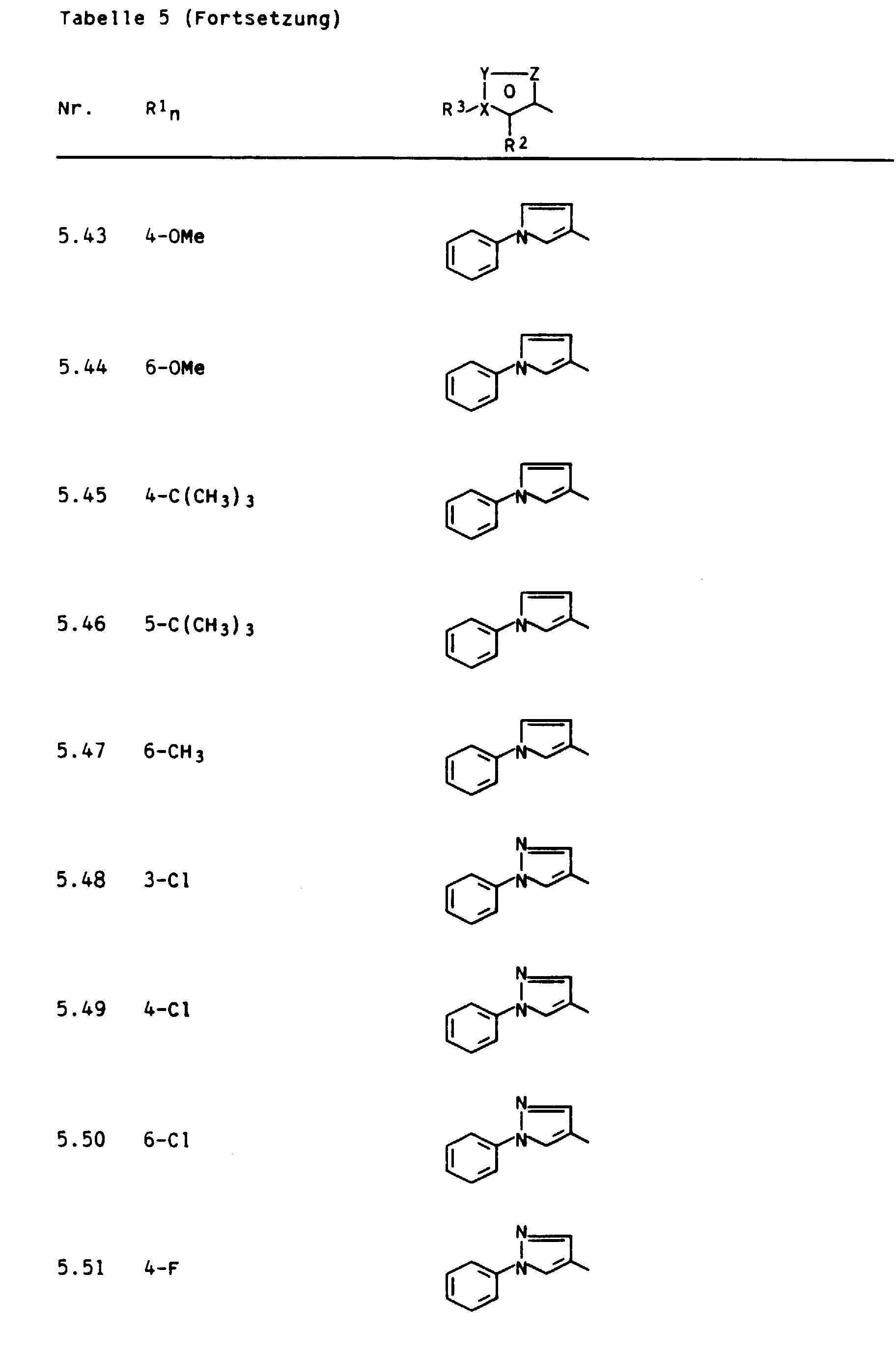

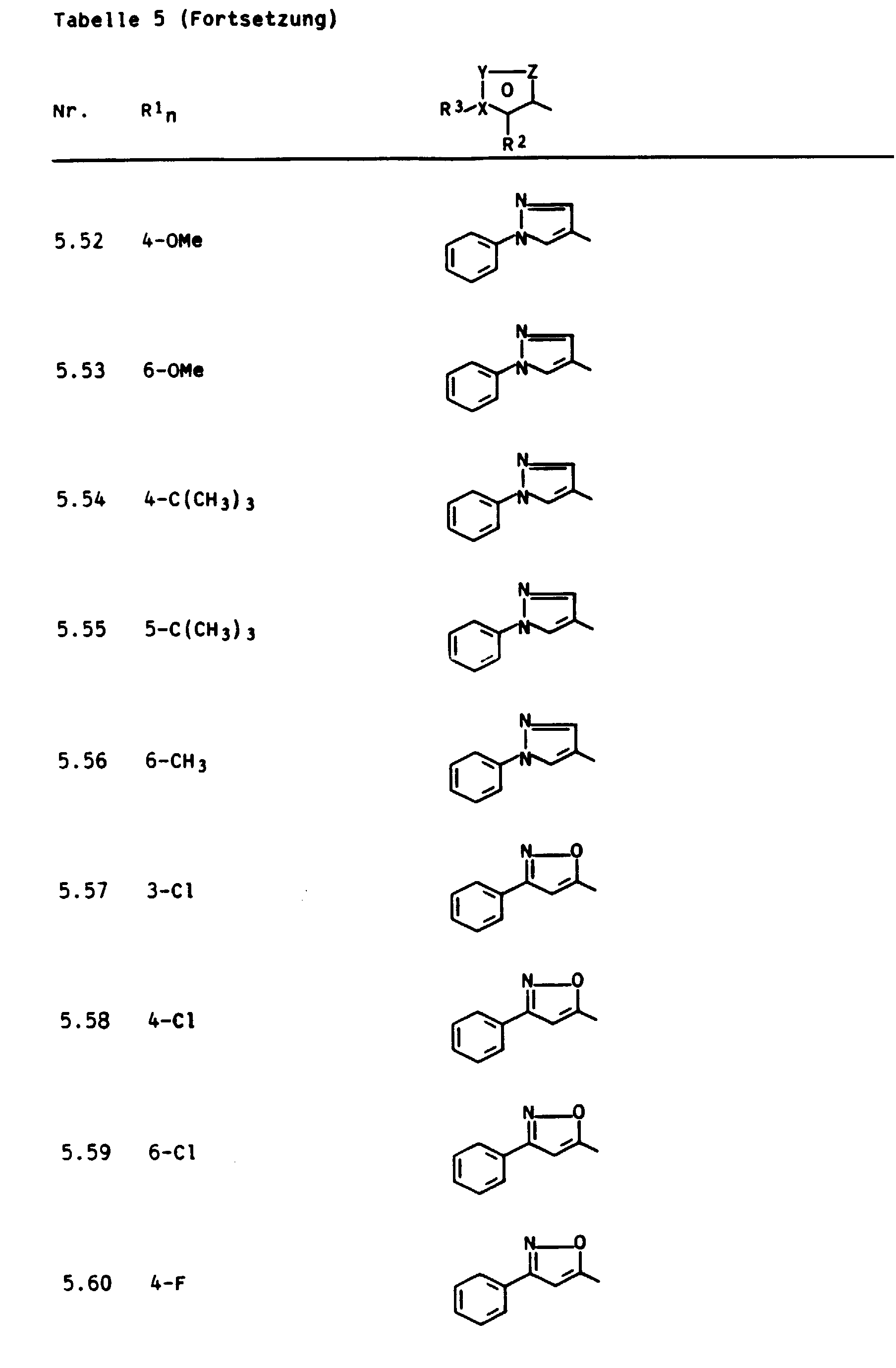

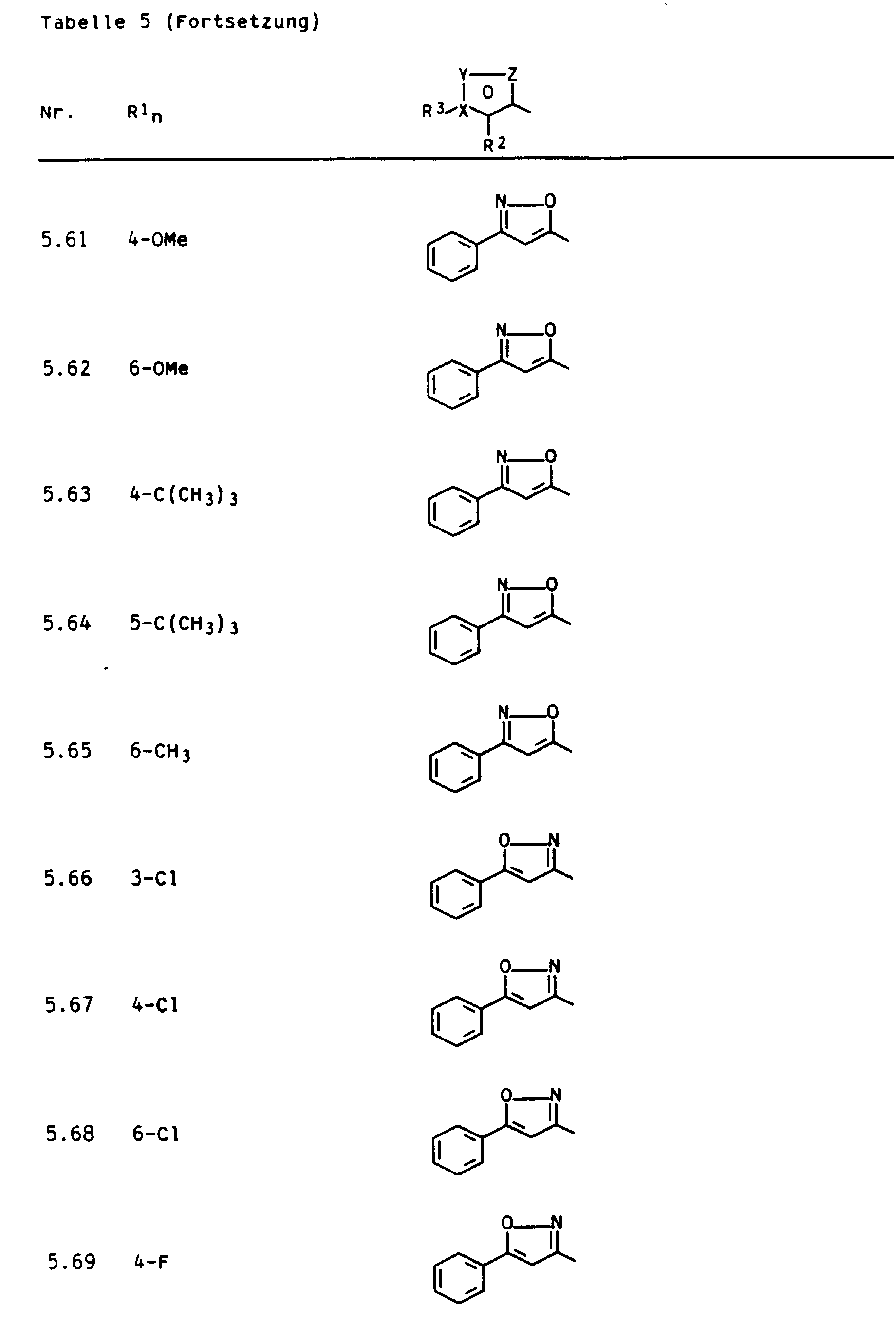

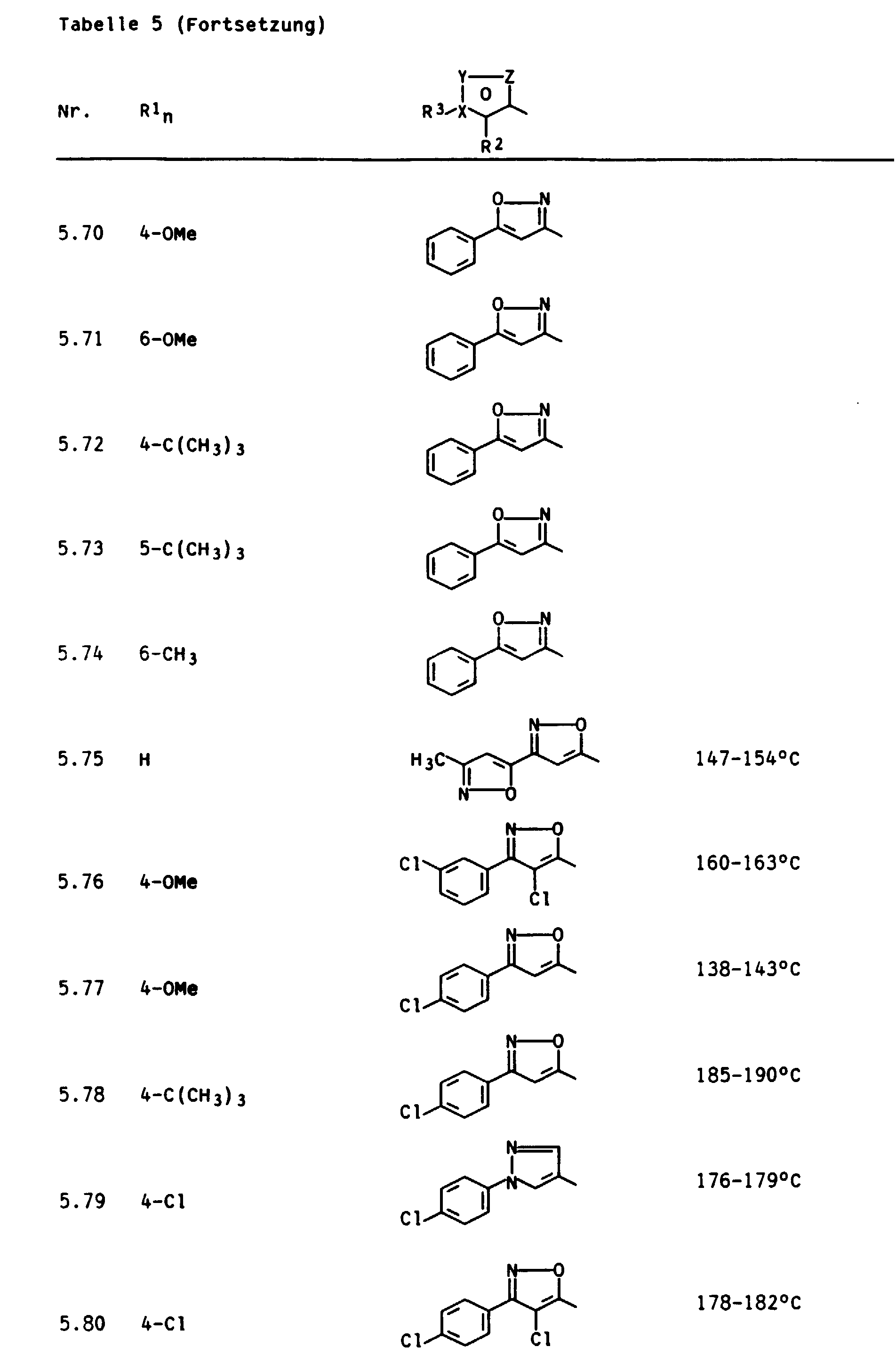

Die neuen Verbindungen eignen sich als Fungizide.

Die neuen Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 1 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 1, 80 Gew.-Teilen Xylol, 10 Gew.-Teilen des Anlagerungsproduktes von 8 bis 10 Mol Ethylenoxid an 1 Mol ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 1, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 1 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1, 10 Gew.-Teilen des Natriumsalzes eines Phenolsulfonsäureharnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
   Dithiocarbamate und deren Derivate, wie
   Ferridimethyldithiocarbamat,
   Zinkdimethyldithiocarbamat,
   Zinkethylenbisdithiocarbamat,
   Manganethylenbisdithiocarbamat,
   Mangan-Zink-ethylendiamin-bis-dithiocarbamat,
   Tetramethylthiuramdisulfide,
   Ammoniak-Komplex von Zink-(N,N-ethylen-bis-dithiocarbamat),
   Ammoniak-Komplex von Zink-(N,N'-propylen-bis-dithiocarbamat),
   Zink-(N,N'-propylen-bis-dithiocarbamat),
   N,N'-Polypropylen-bis-(thiocarbamoyl)-disulfid;
Nitroderivate, wie
   Dinitro-(1-methylheptyl)-phenylcrotonat,
   2-sec-Butyl-4,6-dinitrophenyl-3,3-dimethylacrylat,
   2-sec-Butyl-4,6-dinitrophenyl-isopropylcarbonat,
   5-Nitro-isophthalsäure-di-isopropylester;
heterocyclische Substanzen, wie
   2-Heptadecyl-2-imidazolin-acetat,
   2,4-Dichlor-6-(o-chloranilino)-s-triazin,
   O,O-Diethyl-phthalimidophosphonothioat,
   5-Amino-1-[bis-(dimethylamino)-phosphinyl]-3-phenyl-1,2,4-triazol,
   2,3-Dicyano-1,4-dithioanthrachinon,
   2-Thio-1,3-dithiolo[4,5-b]chinoxalin,
   1-(Butylcarbamoyl)-2-benzimidazol-carbaminsäuremethylester,
   2-Methoxycarbonylamino-benzimidazol,
   2-(Furyl-(2))-benzimidazol,
   2-(Thiazolyl-(4))-benzimidazol,
   N-(1,1,2,2-Tetrachlorethylthio)-tetrahydrophthalimid,
   N-Trichlormethylthio-tetrahydrophthalimid,
   N-Trichlormethylthio-phthalimid,
N-Dichlorfluormethylthio-N',N'-dimethyl-N-phenyl-schwefelsäurediamid,
   5-Ethoxy-3-trichlormethyl-1,2,3-thiadiazol,
   2-Rhodanmethylthiobenzthiazol,
   1,4-Dichlor-2,5-dimethoxybenzol,
   4-(2-Chlorphenylhydrazono)-3-methyl-5-isoxazolon,
   Pyridin-2-thio-1-oxid,
   8-Hydroxychinolin bzw. dessen Kupfersalz,
   2,3-Dihydro-5-carboxanilido-6-methyl-1,4-oxathiin,
   2,3-Dihydro-5-carboxanilido-6-methyl-1,4-oxathiin-4,4-dioxid,
   2-Methyl-5,6-dihydro-4H-pyran-3-carbonsäure-anilid,
   2-Methyl-furan-3-carbonsäureanilid,
   2,5-Dimethyl-furan-3-carbonsäureanilid,
   2,4,5-Trimethyl-furan-3-carbonsäureanilid,
   2,5-Dimethyl-furan-3-carbonsäurecyclohexylamid,
   N-Cyclohexyl-N-methoxy-2,5-dimethyl-furan-3-carbonsäureamid,
   2-Methyl-benzoesäure-anilid,
   2-Iod-benzoesäure-anilid,
   N-Formyl-N-morpholin-2,2,2-trichlorethylacetal,
   Piperazin-1,4-diylbis-(1-(2,2,2-trichlor-ethyl)-formamid,
   1-(3,4-Dichloranilino)-1-formylamino-2,2,2-trichlorethan ,
   2,6-Dimethyl-N-tridecyl-morpholin bzw. dessen Salze,
   2,6-Dimethyl-N-cyclododecyl-morpholin bzw. dessen Salze,
   N-[3-(p-tert.-Butylphenyl)-2-methylpropyl]-cis-2,6-dimethylmorpholin,
   N-[3-(p-tert.-Butylphenyl)-2-methylpropyl]-piperidin,
   1-[2-(2,4-Dichlorphenyl)-4-ethyl-1,3-dioxolan-2-yl-ethyl]-1H-1,2,4-triazol
   1-[2-(2,4-Dichlorphenyl)-4-n-propyl-1,3-dioxolan-2-yl-ethyl]-1H-1,2,4-triazol,
   N-(n-Propyl)-N-(2,4,6-trichlorphenoxyethyl)-N'-imidazol-yl-harnstoff,
   1-(4-Chlorphenoxy)-3,3-dimethyl-1-(1H-1,2,4-triazol-1-yl)-2-butanon,
   1-(4-Chlorphenoxy)-3,3-dimethyl-1-(1H-1,2,4-triazol-1-yl)-2-butanol,
   α-(2-Chlorphenyl)-α-(4-chlorphenyl)-5-pyrimidin-methanol,
   5-Butyl-2-dimethylamino-4-hydroxy-6-methyl-pyrimidin,
   Bis-(p-chlorphenyl)-3-pyridinmethanol,
   1,2-Bis-(3-ethoxycarbonyl-2-thioureido)-benzol,
   1,2-Bis-(3-methoxycarbonyl-2-thioureido)-benzol,
sowie verschiedene Fungizide, wie
   Dodecylguanidinacetat,
   3-[3-(3,5-Dimethyl-2-oxycyclohexyl)-2-hydroxyethyl]-glutarimid, Hexachlorbenzol,
   DL-Methyl-N-(2,6-dimethyl-phenyl)-N-furoyl(2)-alaninat,
   DL-N-(2,6-Dimethyl-phenyl)-N-(2'-methoxyacetyl)-alanin-methylester,
   N-(2,6-Dimethylphenyl)-N-chloracetyl-D,L-2-aminobutyrolacton,
   DL-N-(2,6-Dimethylphenyl)-N-(phenylacetyl)-alaninmethylester,
   5-Methyl-5-vinyl-3-(3,5-dichlorphenyl)-2,4-dioxo-1,3-oxazolidin,
   3-[3,5-Dichlorphenyl(5-methyl-5-methoxymethyl]-1,3-oxazolidin-2,4-dion,
   3-(3,5-Dichlorphenyl)-1-isopropylcarbamoylhydantoin,
   N-(3,5-Dichlorphenyl)-1,2-dimethylcyclopropan-1,2-dicarbonsäureimid,
   2-Cyano-[N-(ethylaminocarbonyl)-2-methoximino]-acetamid,
   1-[2-(2,4-Dichlorphenyl)-pentyl]-1H-1,2,4-triazol,
   2,4-Difluor-α-(1H-1,2,4-triazolyl-1-methyl)-benzhydrylalkohol,
   N-(3-Chlor-2,6-dinitro-4-trifluormethyl-phenyl)-5-trifluormethyl-3-chlor-2-aminopyridin,
   1-((bis-(4-Fluorphenyl)-methylsilyl)-methyl)-1H-1,2,4-triazol.

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Nephotettix cincticeps, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

### Anwendungsbeispiele

Als bekannte Vergleichswirkstoffe wurden die in EP 378 755 beschriebenen Verbindungen Nr. 121 (A), Nr. 279 (B), Nr. 147 (C), Nr. 123 (D) und Nr. 125 (E) verwendet.

### Anwendungsbeispiel 1

### Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Verbindungen Nr. 3.13, 3.17, 4.22, 4.23 und 3.23 bei der Anwendung als 250 ppm Wirkstoff enthaltende wäßrige Spritzbrühen eine bessere fungizide Wirkung haben (90 %) als die bekannten Wirkstoffe A, B und C (60 %).

### Anwendungsbeispiel 2

### Wirksamkeit gegen Septoria nodorum

Blätter von in Töpfen gewachsenen Weizenkeimlingen der Sorte "Jubilar" wurden mit wäßriger Spritzbrühe, die 80 % Wirkstoff und 20 % Emulgiermittel in der Trockensubstanz enthielt, tropfnaß besprüht. Am folgenden Tag wurden die angetrockneten Pflanzen mit einer wäßrigen Sporensuspension von Septoria nodorum infiziert und dann für 7 Tage bei 17-19°C und 95 % relativer Luftfeuchtigkeit weiter kultiviert. Das Ausmaß des Pilzbefalls wurde dann visuell ermittelt.

Das Ergebnis zeigt, daß die Verbindungen Nr. 3.31, Nr. 3.17 und Nr. 3.18 bei der Anwendung als 500 ppm Wirkstoff enthaltende wäßrige Spritzbrühe eine bessere fungizide Wirkung haben (95 %) als die bekannten Wirkstoffe D, B und E (60 %).

## Patentansprüche

1. α-Arylacrylsäurederivate der allgemeinen Formel I 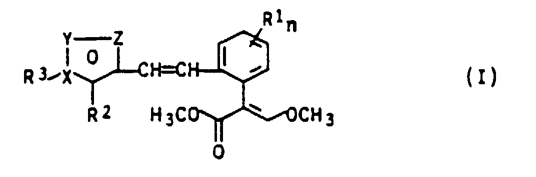 in der die Substituenten folgende Bedeutung haben:
X C, N
Y CR⁴, N, O, S
Z CR⁵, N, O, S, wobei Y und Z nicht gleichzeitig O, S oder O und S bedeuten,
n 0 bis 4
R¹ Nitro, Cyano, Halogen,
C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio,
oder, für den Fall, daß n 2, 3 oder 4 bedeutet, zwei benachbarte Substituenten R¹ zusammen eine 1,3-Butadien-1,4-diylgruppe bedeuten, welche ein bis vier Halogenatome und oder ein oder zwei der folgenden Gruppen tragen kann: Nitro, Cyano, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
R² Cyano, Dimethylamino, Nitro, Halogen, C₁-C₄-Alkyl; partiell oder vollständig halogeniertes C₁-C₄-Alkyl, C₁-C₄-Alkoxycarbonyl,
R² bedeutet zusätzlich Wasserstoff, wobei dann R³ einen heterocyclischen oder heteroaromatischen Rest bedeutet oder R⁴ oder R⁵ verschieden von Wasserstoff sind;
R³ Wasserstoff;
Alkyl mit bis zu 12 Kohlenstoffatomen, welches partiell oder vollständig halogeniert sein kann und welches desweiteren einen bis vier der folgenden Reste tragen kann: Cyano, Cyanato, Thiocyanato, Nitro und C₁-C₆-Alkoxy;
C₃-C₈-Cycloalkyl, C₅-C₈₋Cycloalkenyl, C₅-C₈-Cycloalkdienyl oder ein 3- bis 6-gliedriger, gesättigter oder partiell ungesättigter Heterocyclus, welcher ein bis drei Heteroatome, ausgewählt aus einer Gruppe bestehend aus zwei Stickstoffatomen, zwei Sauerstoff- und zwei Schwefelatomen oder aus einer Gruppe bestehend aus drei Sauerstoff- und Schwefelatomen, enthält, wobei diese cyclischen Gruppen noch einen bis drei der folgenden Reste tragen können: Cyano, Dimethylamino, Nitro, Halogen, C₁-C₆-Alkyl, C₁-C₄-Alkoxy, C₁-C₄-Alkylthio und C₃-C₈-Cycloalkyl, und wobei die Heterocyclen noch zusätzlich soviele Halogenatome tragen können, daß die Gesamtzahl der Reste 4 oder 5 beträgt;
oder ein ein- oder zweikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann, wobei diese Gruppen ein bis fünf Halogenatome und/oder ein bis vier der folgenden Reste tragen können: Cyano, Cyanato, Thiocyanato, Nitro, Amino, Hydroxy, Carboxyl, C₁-C₆-Alkyl, partiell oder vollständig halogeniertes C₁-C₄-Alkyl, C₁-C₆-Alkoxy, partiell oder vollständig halogeniertes C₁-C₄-Alkoxy, C₁-C₆-Alkylthio, C₃-C₈-Cycloalkyl, C₁-C₆-Alkoxycarbonyl, Phenyl oder Phenoxy, wobei die letztgenannten Phenylringe ihrerseits ein bis drei der folgenden Substituenten tragen können: Cyano, Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy, und C₁-C₄-Alkylthio, und wobei diese Phenylringe zusätzlich noch soviele Halogenatome tragen können, daß die Gesamtzahl der Reste 4 oder 5 beträgt;
R⁴,R⁵ Wasserstoff, Cyano, Dimethylamino, Nitro, C₁-C₄-Alkyl, partiell oder vollständig halogeniertes C₁-C₄-Alkyl, C₁-C₆-Alkoxycarbonyl.

2. Verfahren zur Herstellung der Verbindungen der Formel I gemäß Anspruch 1, dadurch gekennzeichnet, daß man ein Triphenylphosphoniumsalz oder einen Phosphonsäureester der allgemeinen Formel II, 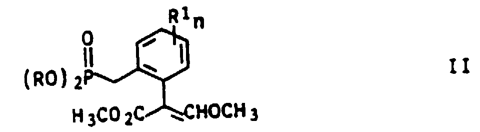 in der R für eine C₁-C₈-Alkylgruppe steht, in einem inerten organischen Lösungsmittel in Gegenwart einer Base mit einem Aldehyd der allgemeinen Formel III, 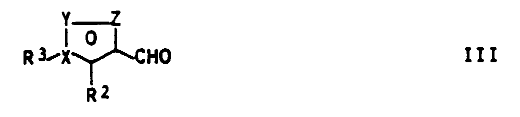 umsetzt.

3. Verfahren zur Herstellung der Verbindungen der Formel I gemäß Anspruch 1, dadurch gekennzeichnet, daß man ein Triphenylphosphoniumsalz oder einen Phosphonsäureester der allgemeinen Formel IV, 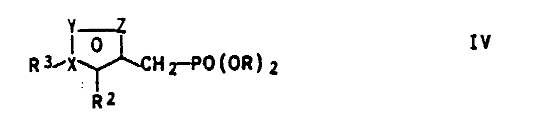 in der R für eine C₁-C₈-Alkylgruppe steht, in einem inerten organischen Lösungsmittel in Gegenwart einer Base mit dem Benzaldehyd der Formel V, 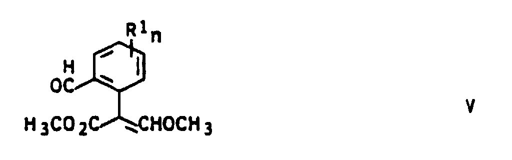 umsetzt.

4. Schädlingsbekämpfungsmittel und fungizide Mittel, enthaltend eine wirksame Menge eines α-Arylacrylsäurederivats der allgemeinen Formel I gemäß Anspruch 1 und einen inerten Zusatzstoff.

5. Verfahren zur Bekämpfung von Schädlingen aus der Klasse der Insekten, Spinnentiere und Nematoden, dadurch gekennzeichnet, daß man die Schädlinge oder ihren Lebensraum mit einer wirksamen Menge eines α-Arylacrylsäurederivats der allgemeinen Formel I gemäß Anspruch 1 behandelt.

6. Verbindungen der Formel I gemäß Anspruch 1, in der n den Wert 0 hat, R² Chlor, R³ gegebenenfalls substituiertes Phenyl, X C, Y N und Z O bedeuten.

7. Verbindungen der Formel I gemäß Anspruch 1, in der n den Wert 0 hat, R² Trifluormethyl, R³ gegebenenfalls substituiertes Phenyl, X N, Y N und Z CH bedeuten.

8. Verbindungen der Formel I gemäß Anspruch 1, in der n den Wert O hat, R² Methyl, R³ gegebenenfalls substituiertes Phenyl, X C, Y O und Z N bedeuten.

9. Verfahren zur Bekämpfung von Pilzen im Pflanzen- und Materialschutz, dadurch gekennzeichnet, daß man die Pilze oder ihren Lebensraum mit einer wirksamen Menge eines α-Arylacrylsäurederivats der allgemeinen Formel I gemäß Anspruch 1 behandelt.

## Claims

1. An α-arylacrylic acid derivative of the formula I 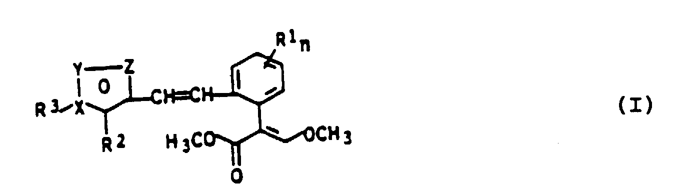 where
X is C or N,
Y is CR⁴, N, O or S,
Z is CR⁵, N, O or S, Y and Z not simultaneously being O, S or O and S,
n is from 0 to 4,
R¹ is nitro, cyano, halogen,
C₁-C₄-alkyl, partially or completely halogenated C₁-C₄-alkyl, C₁-C₄-alkoxy, partially or completely halogenated C₁-C₄-alkoxy or C₁-C₄-alkylthio,
or, where n is 2, 3 or 4, two adjacent substituents R¹ together form 1,3-butadiene-1,4-diyl, which may carry from one to four halogen atoms and/or one or two of the following groups: nitro, cyano, C₁-C₄-alkyl, partially or completely halogenated C₁-C₄-alkyl, C₁-C₄-alkoxy, partially or completely halogenated C₁-C₄-alkoxy or C₁-C₄-alkylthio;
R² is cyano, dimethylamino, nitro, halogen, C₁-C₄-alkyl, partially or completely halogenated C₁-C₄-alkyl or C₁-C₄-alkoxycarbonyl,
R² is additionally hydrogen, in which case R³ is a heterocyclic or heteroaromatic radical or R⁴ or R⁵ is not hydrogen;
R³ is hydrogen;
alkyl having up to 12 carbon atoms, which may be partially or completely halogenated and may additionally carry from one to four of the following radicals: cyano, cyanato, thiocyanato, nitro and C₁-C₆-alkoxy;
C₃-C₈-cycloalkyl, C₅-C₈-cycloalkenyl, C₅-C₈-cycloalkdienyl or a 3- to 6-membered saturated or partially unsaturated heterocycle containing from one to three hetero atoms selected from a group consisting of two nitrogen atoms, two oxygen atoms and two sulfur atoms or from a group consisting of three oxygen and sulfur atoms, it being possible for these cyclic groups to carry additionally from one to three of the following radicals: cyano, dimethylamino, nitro, halogen, C₁-C₆-alkyl, C₁-C₄-alkoxy, C₁-C₄-alkylthio and C₃-C₈-cycloalkyl, where the heterocycles may additionally carry a sufficient number of halogen atoms for the total number of radicals to be 4 or 5;
or a mononuclear or dinuclear aromatic system which, in addition to carbon atoms, may contain from one to four nitrogen atoms or from one to three hetero atoms selected from a group consisting of two nitrogen atoms and one oxygen or sulphur atom, it being possible for these groups to carry from one to five halogen atoms and/or from one to four of the following radicals: cyano, cyanato, thiocyanato, nitro, amino, hydroxyl, carboxyl, C₁-C₆-alkyl, partially or completely halogenated C₁-C₄-alkyl, C₁-C₆-alkoxy, partially or completely halogenated C₁-C₄-alkoxy, C₁-C₆-alkylthio, C₃-C₈-cycloalkyl, C₁-C₆-alkoxycarbonyl, phenyl or phenoxy, where the last phenyl rings in turn may carry from one to three of the following substituents: cyano, nitro, halogen, C₁-C₄-alkyl, partially or completely halogenated C₁-C₄-alkyl, C₁-C₄-alkoxy, partially or completely halogenated C₁-C₄-alkoxy and C₁-C₄-alkylthio, and where these phenyl rings may additionally carry a sufficient number of halogen atoms for the total number of radicals to be 4 or 5;
R⁴ and R⁵ are each hydrogen, cyano, dimethylamino, nitro, C₁-C₄-alkyl, partially or completely halogenated C₁-C₄-alkyl or C₁-C₆-alkoxycarbonyl.

2. A process for the preparation of a compound of the formula I as claimed in claim 1, wherein a triphenylphosphonium salt or a phosphonic ester of the formula II 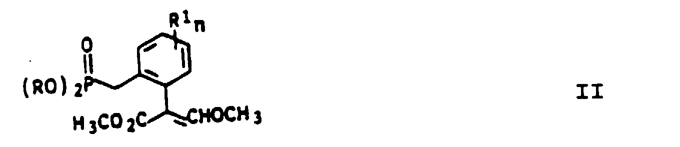 where R is C₁-C₈-alkyl, is reacted with an aldehyde of the formula III 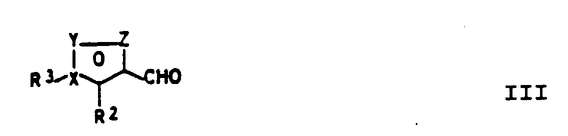 in an inert organic solvent in the presence of a base.

3. A process for the preparation of a compound of the formula I as claimed in claim 1, wherein a triphenylphosphonium salt or a phosphonic ester of the formula IV 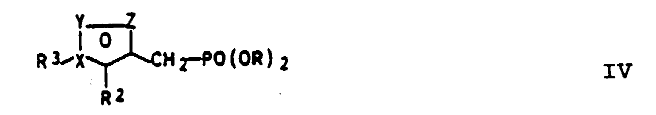 where R is C₁-C₈-alkyl, is reacted with the benzaldehyde of the formula V 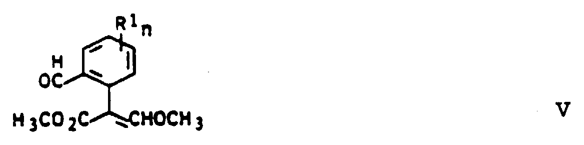 in an inert organic solvent in the presence of a base.

4. A pesticide or fungicide containing an effective amount of an α-arylacrylic acid derivative of the formula I as claimed in claim 1 and an inert additive.

5. A method for controlling pests from the class of the insects, arachnids and nematodes, wherein the pests or their habitat is or are treated with an effective amount of an α-arylacrylic acid derivative of the formula I as claimed in claim 1.

6. A compound of the formula I as claimed in claim 1, wherein n is 0, R² is chlorine, R³ is unsubstituted or substituted phenyl, X is C, Y is N and Z is O.

7. A compound of the formula I as claimed in claim 1,wherein n is 0, R² is trifluoromethyl, R³ is unsubstituted or substituted phenyl, X and Y are each N and Z is CH.

8. A compound of the formula I as claimed in claim 1, wherein n is 0, R² is methyl, R³ is unsubstituted or substituted phenyl, X is C, Y is O and Z is N.

9. A method for controlling fungi in crop and material protection, wherein the fungi or their habitat is or are treated with an effective amount of an α-arylacrylic acid derivative of the formula I as claimed in claim 1.

## Revendications

1. Dérivés d'acides alpha-arylacryliques de formule générale I 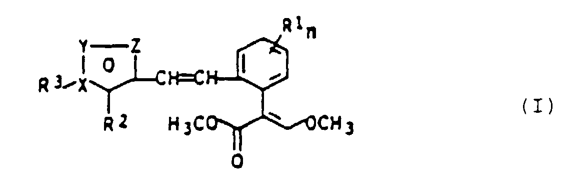 dans laquelle les symboles ont les significations suivantes :
X : C, N
Y : CR⁴, N, O, S
Z : CR⁵, N, O, S, sous réserve que Y et Z ne peuvent représenter simultanément O, S ou O et S,
n est un nombre allant de 0 à 4,
R¹ représente un goupe nitro, cyano, un halogène,
un groupe alkyle en C1-C4, alkyle en C1-C4 partiellement ou totalement halogéné, alcoxy en C1-C4, alcoxy en C1-C4 partiellement ou totalement halogéné ou alkylthio en C1-C4,
ou bien, lorsque n est égal à 2, 3 ou 4, deux substituants R¹ voisins forment ensemble un groupe 1,3-butadiène-1,4-diyle qui peut porter un à quatre atomes d'halogènes ou un ou deux des substituants suivants : nitro, cyano, alkyle en C1-C4, alkyle en C1-C4 partiellement ou totalement halogéné, alcoxy en C1-C4, alcoxy en C1-C4 partiellement ou totalement halogéné ou alkylthio en C1-C4 ;
R²: un groupe cyano, diméthylamino, nitro, un halogène, un groupe alkyle en C1-C4, alkyle en C1-C4 partiellement ou totalement halogéné, (alcoxy en C1-C4)carbonyle,
R²: peut en outre représenter l'hydrogène, et alors R³ représente un radical hétérocyclique ou hétéroaromatique ou bien R⁴ ou R⁵ a une signification autre que l'hydrogène ;
R³: l'hydrogène ;
un groupe alkyle contenant jusqu'à douze atomes de carbone, qui peut être partiellement ou totalement halogéné et qui peut en outre porter un à quatre des substituants suivants : cyano, cyanato, thiocyanato, nitro et alcoxy en C1-C6 ;
un groupe cycloalkyle en C3-C8, cycloalcényle en C5-C8, cycloalcadiényle en C5-C8 ou un hétérocycle de trois à six chaînons, saturé ou partiellement insaturé, qui contient un à trois hétéroatomes choisis dans un groupe consistant en deux atomes d'azote, deux atomes d'oxygène et deux atomes de soufre ou dans un groupe consistant en trois atomes d'oxygène et de soufre, ces groupes cycliques pouvant encore porter un à trois des substituants suivants : cyano, diméthylamino, nitro, halogéno, alkyle en C1-C6, alcoxy en C1-C4, alkylthio en C1-C4 et cycloalkyle en C3-C8, les hétérocycles pouvant encore porter des atomes d'halogènes en nombre tel que le nombre total des substituants soit de 4 ou 5 :
ou un système aromatique mono- ou bi-cyclique qui, en plus des atomes de carbone, peut contenir un à quatre atomes d'azote ou un à trois hétéroatomes choisis dans un groupe consistant en deux atomes d'azote et un atome d'oxygène ou de soufre, ces groupes pouvant porter un à cinq atomes d'halogènes et/ou un à quatre des substituants suivants : cyano, cyanato, thiocyanato, nitro, amino, hydroxy, carboxyle, alkyle en C1-C6, alkyle en C1-C4 partiellement ou totalement halogéné, alcoxy en C1-C6, alcoxy en C1-C4 partiellement ou totalement halogéné, alkylthio en C1-C6, cycloalkyle en C3-C8, (alcoxy en C1-C6)carbonyle, phényle ou phénoxy, les cycles phényle mentionnés en dernier pouvant eux-mêmes porter un à trois des substituants suivants : cyano, nitro, halogéno, alkyle en C1-C4, alkyle en C1-C4 partiellement ou totalement halogéné, alcoxy en C1-C4, alcoxy en C1-C4 partiellement ou totalement halogéné et alkylthio en C1-C4, et ces cycles phényle pouvant encore porter des atomes d'halogènes en nombre tel que le nombre total des substituants soit de 4 ou 5 ;
R⁴, R⁵ : l'hydrogène, un groupe cyano, diméthylamino, nitro, alkyle en C1-C4, alkyle en C1-C4 partiellement ou totalement halogéné, (alcoxy en C1-C6)carbonyle.

2. Procédé de préparation des composés de formule I selon la revendication 1, caractérisé par le fait que l'on fait réagir un sel de triphénylphosphonium ou un ester phosphonique de formule générale II 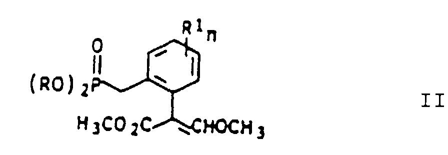 dans laquelle R représente un groupe alkyle en C1-C8, dans un solvant inerte et en présence d'une base, avec un aldéhyde de formule générale III

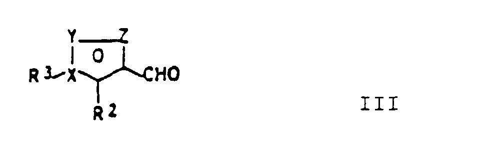

3. Procédé de préparation des composés de formule I selon la revendication 1, caractérisé par le fait que l'on fait réagir un sel de triphénylphosphonium ou un ester phosphonique de formule générale IV 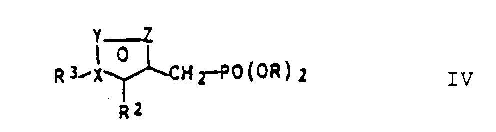 dans laquelle R représente un groupe alkyle en C1-C8, dans un solvant organique et en présence d'une base, avec le benzaldéhyde de formule V

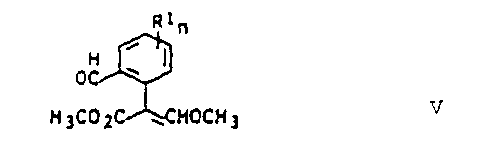

4. Produits parasiticides et produits fongicides contenant une quantité efficace d'un dérivé d'acide alpha-arylacrylique de formule générale I selon revendication 1 et un additif inerte.

5. Procédé pour combattre les parasites des classes des insectes, des arachnides et des nématodes, caractérisé par le fait que l'on traite les parasites ou leur habitat par une quantité efficace d'un dérivé d'acide alpha-arylacrylique de formule générale I selon revendication 1.

6. composés de formule I selon la revendication 1 dans laquelle n est égal à 0, R² représente le chlore, R³ un groupe phényle éventuellement substitué, X représente C, Y représente N et Z représente 0.

7. Composés de formule I selon la revendication 1 dans laquelle n est égal à 0, R² représente un groupe trifluorométhyle, R³ un groupe phényle éventuellement substitué, X représente N, Y représente N et Z représente CH.

8. Composés de formule I selon la revendication 1 dans laquelle n est égal à 0, R² représente un groupe méthyle, R³ un groupe phényle éventuellement substitué, X représente C, Y représente O et Z représente N.

9. Procédé pour combattre les mycètes en vue de protéger des végétaux et des matières, caractérisé par le fait que l'on traite les mycètes ou leur habitat par une quantité efficace d'un dérivé d'acide alpha-arylacrylique de formule générale I selon la revendication 1.
